# EUROPEAN PATENT APPLICATION

(11) **EP 4 844 781 A1**
(43) Date of publication of application: **25.11.2026**
(21) Application number: 26175828.8
(22) Date of filing: 30.04.2026
(51) Int. Cl.: G16H 50/50, G16H 20/40

(54) **ADJUSTING TRANSFORMATION OF DIGITAL DENTAL MODEL**

(30) Priority: 21.05.2025 US 202519214825
(71) Applicant: exocad GmbH, 64295 Darmstadt (DE)
(72) Inventor: CHIOSA, Iurie, 64331 Weiterstadt (DE); GERTH, Maik, 64342 Seeheim-Jugenheim (DE); STEINBRECHER, Tillmann, 64342 Seeheim-Jugenheim (DE)
(74) Representative: Richardt Patentanwälte PartG mbB

(57) **Abstract**

Disclosed is a computer-implemented method for adjusting a transformation of a first three-dimensional digital dental model. The transforming comprises a positioning of the first three-dimensional digital dental model (100) relative to a second three-dimensional digital dental model. The positioning of the first three-dimensional digital dental model (100) relative to the second three-dimensional digital dental model (120) is restricted by a boundary condition (122) which results in a rotating of the first three-dimensional digital dental model. A definition of the start orientation (110) of the first three-dimensional digital dental model (100) is stored for further use. The transforming of the first three-dimensional digital dental model (100) is adjusted. A preserving of the boundary condition (122) by the adjusting results in a further rotating of the first three-dimensional digital dental model (100) using the stored definition of the start orientation (110) as a first reference orientation. The further rotating at least partially reverses the rotating of the first three-dimensional digital dental model.

## Description

### FIELD OF THE INVENTION

The invention relates to the field of dental technology. More particularly, the invention relates to a method, a computer program product, and a computer device for adjusting a transformation of a first three-dimensional digital dental model of a first dental structure relative to a second three-dimensional digital dental model of a second dental structure.

### BACKGROUND

In modern dental technology, computer-based approaches are used for configuring and manufacturing dental prostheses. For configuring such dental prostheses, a precise transforming, e.g., positioning of dental structures for the dental prostheses may be required. In the course of the positioning, adjustments of the dental structures may become necessary. Implementing such adjustments may be a challenging and work-intensive task.

### SUMMARY OF THE INVENTION

In one aspect, a computer-implemented method is disclosed for adjusting a transformation of a first three-dimensional digital dental model of a first dental structure relative to a second three-dimensional digital dental model of a second dental structure. The method comprises receiving the first three-dimensional digital dental model. The second three-dimensional digital dental model is received. The first three-dimensional digital dental model is transformed. The transforming comprises a positioning of the first three-dimensional digital dental model relative to the second three-dimensional digital dental model. The positioning of the first three-dimensional digital dental model relative to the second three-dimensional digital dental model is restricted by a boundary condition defining one or more distance criteria to be preserved between the first and second three-dimensional digital dental model. A preserving of the one or more distance criteria by the positioning results in a rotating of the first three-dimensional digital dental model around an axis of rotation increasing an angle of a current orientation of the first three-dimensional digital dental model relative to an initial start orientation of the first three-dimensional digital dental model at a start of the rotation until the current orientation reaches a provisional end orientation of the first three-dimensional digital dental model at an end of the rotation with a maximum angle relative to the start orientation. A definition of the start orientation is stored for further use.

The method further comprises adjusting the transforming of the first three-dimensional digital dental model. A preserving of the one or more distance criteria by the adjusting of the transforming results in a further rotating of the first three-dimensional digital dental model around the axis of rotation using the stored definition of the start orientation as a first reference orientation. The further rotating at least partially reverses the rotating of the first three-dimensional digital dental model such that the current angle relative to the start orientation is decreased.

In another aspect, a computer program product is disclosed for adjusting a transformation of a first three-dimensional digital dental model of a first dental structure relative to a second three-dimensional digital dental model of a second dental structure. The computer program product comprises a non-transitory computer-readable storage medium having program instructions embodied therewith.

The program instructions are executable by a processing unit of a computer device to cause the computer device to receive the first three-dimensional digital dental model. The second three-dimensional digital dental model is received. The first three-dimensional digital dental model is transformed. The transforming comprises a positioning of the first three-dimensional digital dental model relative to the second three-dimensional digital dental model. The positioning of the first three-dimensional digital dental model relative to the second three-dimensional digital dental model is restricted by a boundary condition defining one or more distance criteria to be preserved between the first and second three-dimensional digital dental model. A preserving of the one or more distance criteria by the positioning results in a rotating of the first three-dimensional digital dental model around an axis of rotation increasing an angle of a current orientation of the first three-dimensional digital dental model relative to an initial start orientation of the first three-dimensional digital dental model at a start of the rotation until the current orientation reaches a provisional end orientation of the first three-dimensional digital dental model at an end of the rotation with a maximum angle relative to the start orientation. A definition of the start orientation is stored for further use.

The program instructions are further executable by the processing unit of the computer device to cause the computer device to adjust the transforming of the first three-dimensional digital dental model. A preserving of the one or more distance criteria by the adjusting of the transforming results in a further rotating of the first three-dimensional digital dental model around the axis of rotation using the stored definition of the start orientation as a first reference orientation. The further rotating at least partially reverses the rotating of the first three-dimensional digital dental model such that the current angle relative to the start orientation is decreased.

In another aspect, a computer device is disclosed for adjusting a transformation of a first three-dimensional digital dental model of a first dental structure relative to a second three-dimensional digital dental model of a second dental structure. The computer device comprises a processing unit and a memory unit storing program instructions executable by the processing unit.

An execution of the program instructions by the processing unit causes the computer device to receive the first three-dimensional digital dental model. The second three-dimensional digital dental model is received. The first three-dimensional digital dental model is transformed. The transforming comprises a positioning of the first three-dimensional digital dental model relative to the second three-dimensional digital dental model. The positioning of the first three-dimensional digital dental model relative to the second three-dimensional digital dental model is restricted by a boundary condition defining one or more distance criteria to be preserved between the first and second three-dimensional digital dental model. A preserving of the one or more distance criteria by the positioning results in a rotating of the first three-dimensional digital dental model around an axis of rotation increasing an angle of a current orientation of the first three-dimensional digital dental model relative to an initial start orientation of the first three-dimensional digital dental model at a start of the rotation until the current orientation reaches a provisional end orientation of the first three-dimensional digital dental model at an end of the rotation with a maximum angle relative to the start orientation. A definition of the start orientation is stored for further use.

The program instructions are further executable by the processing unit of the computer device to cause the computer device to adjust the transforming of the first three-dimensional digital dental model. A preserving of the one or more distance criteria by the adjusting of the transforming results in a further rotating of the first three-dimensional digital dental model around the axis of rotation using the stored definition of the start orientation as a first reference orientation. The further rotating at least partially reverses the rotating of the first three-dimensional digital dental model such that the current angle relative to the start orientation is decreased.

### BRIEF DESCRIPTION OF THE DRAWINGS

In the following, examples are described in greater detail making reference to the drawings in which:
Fig. 1 is a flowchart of an exemplary method for adjusting a transformation of a first three-dimensional digital dental model relative to a second three-dimensional digital dental model;
Fig. 2 is a flowchart of an exemplary method for manufacturing a dental prosthesis using an adjusted three-dimensional digital dental model;
Fig. 3 illustrates a first three-dimensional digital dental model with a start orientation and a second three-dimensional digital dental model;
Fig. 4 illustrates the start orientation of the first three-dimensional digital dental model for a positioning;
Fig. 5 illustrates a provisional end orientation of the first three-dimensional digital dental model reached by the positioning;
Fig. 6 illustrates an adjusting of the transforming of the first three-dimensional digital dental model;
Fig. 7 illustrates an adjusting of the transforming of the first three-dimensional digital dental model;
Fig. 8 illustrates an adjusting of the transforming of the first three-dimensional digital dental model;
Fig. 9 illustrates visual indicators displayed during the adjusting of the transforming of the first three-dimensional digital dental model;
Fig. 10 is a schematic diagram of an exemplary computer device for adjusting a transformation of a first three-dimensional digital dental model relative to a second three-dimensional digital dental model;
Fig. 11 shows an exemplary computer system with a computer device for adjusting a transformation of a first three-dimensional digital dental model relative to a second three-dimensional digital dental model; and
Fig. 12 shows an exemplary manufacturing system for manufacturing a dental prosthesis using an adjusted three-dimensional digital dental model.

### DETAILED DESCRIPTION

The objectives underlying the invention are solved by the features of the independent claims.

It is understood that one or more of the following examples may be combined as long as the combined examples are not mutually exclusive.

For example, a computer-implemented method is provided for adjusting a transformation of a first three-dimensional digital dental model of a first dental structure relative to a second three-dimensional digital dental model of a second dental structure. The method comprises receiving the first three-dimensional digital dental model. The second three-dimensional digital dental model is received. The first three-dimensional digital dental model is transformed. The transforming comprises a positioning of the first three-dimensional digital dental model relative to the second three-dimensional digital dental model. The positioning of the first three-dimensional digital dental model relative to the second three-dimensional digital dental model is restricted by a boundary condition defining one or more distance criteria to be preserved between the first and second three-dimensional digital dental model. A preserving of the one or more distance criteria by the positioning results in a rotating of the first three-dimensional digital dental model around an axis of rotation increasing an angle of a current orientation of the first three-dimensional digital dental model relative to an initial start orientation of the first three-dimensional digital dental model at a start of the rotation until the current orientation reaches a provisional end orientation of the first three-dimensional digital dental model at an end of the rotation with a maximum angle relative to the start orientation. A definition of the start orientation is stored for further use.

The method further comprises adjusting the transforming of the first three-dimensional digital dental model, in particular the positioning of the first three-dimensional digital dental model. A preserving of the one or more distance criteria by the adjusting of the transforming results in a further rotating of the first three-dimensional digital dental model around the axis of rotation using the stored definition of the start orientation as a first reference orientation. The further rotating at least partially reverses the rotating of the first three-dimensional digital dental model such that the current angle relative to the start orientation is decreased.

Examples may have the beneficial effect that a consistent and reproducible relative positioning, e.g., alignment of the first and second three-dimensional digital dental models, is achievable. For this purpose, the stored start orientation may be used as a fixed reference for adjustments to the rotations under the preserved distance criteria.

Examples may have the beneficial effect that fine-grained, distance-aware corrections may be achievable. This may, e.g., be based on the method's capability to at least partially reverse the initial rotation around the axis of rotation while enforcing the distance criteria, i.e., the boundary condition as a constraint for the transforming and adjusting.

Examples may have the beneficial effect of facilitating and/or improving a positioning, i.e., placing of the first three-dimensional digital dental model relative to the second three-dimensional digital dental model. In particular, a reversal of a rotation executed during the positioning by a back rotation may be facilitated. The back rotation is a back rotation relative to the rotation executed during the positioning, which is referred to as a forward rotation. Examples may enable the back rotation during the adjusting to follow exactly the same path of rotation as the forward rotation during positioning.

The transforming of the first three-dimensional digital dental model may, e.g., comprise a positioning of the first three-dimensional digital dental model. A positioning of the first three-dimensional digital dental model may, e.g., comprise a translational moving of the first three-dimensional digital dental model and/or a rotating of the first three-dimensional digital dental model. The transforming of the first three-dimensional digital dental model may, e.g., comprise a scaling of the first three-dimensional digital dental model.

For example, the further rotating resulting from the adjusting of the transforming comprises a back rotation. For example, the back rotation results in a back correction, which at least partly reverses the positioning. For example, the further rotating resulting from the adjusting of the transforming comprises a forward rotation. For example, the forward rotation results in a forward correction, which at least partly restores the positioning.

For example, the adjusting may be used to partially undo the rotation of the positioning. For example, the adjusting may be used to completely undo the rotation of the positioning. In this case, the further rotating may completely reverse the rotating of the first three-dimensional digital dental model executed during the positioning such that the current angle relative to the start orientation is decreased to zero. At the end of such an adjusting, the first three-dimensional digital dental model may reach the start orientation.

Examples may have the beneficial effect that, e.g., unwanted rotations of the first three-dimensional digital dental model occurring during the positioning of the first three-dimensional digital dental model may effectively and precisely be undone. Such unwanted rotations may, e.g., result from a preserving of the distance criteria during the positioning. The preserving of the distance criteria may result in a rotating, which is to be undone. Storing and using the start orientation may facilitate the reversing. This may, e.g., be particularly beneficial, in case the positioning comprises a combination of a translational movement and the rotating of the first three-dimensional digital dental model of which only the rotating is to be reversed partially or completely.

For example, definitions of the start orientation and/or the provisional end orientation of the first three-dimensional digital dental model during the positioning may be stored. Visual indicators, like arrows, may be displayed and used for indicating these reference orientations using the stored definitions. Thus, the start orientation and/or the provisional end orientation may be used as reference orientations between which the adjusting is executed. Furthermore, a visual indicator of a current orientation of the first three-dimensional digital dental model may be displayed, e.g., in form of an arrow. Thus, throughout the adjusting a precise relation of the current orientation of the first three-dimensional digital dental model to the start and/or provisional end orientation may be assessable. Using this assessability, the first three-dimensional digital dental model may be precisely arranged in a desired orientation relative to the start and/or the provisional end orientation. A precise indication of the start orientation and/or the provisional end orientation during the adjusting may even enable a returning to the precise start orientation and/or the provisional end orientation during the adjusting.

For example, the rotating resulting from the preserving of the one or more distance criteria, i.e., the boundary condition, may result from resolving or preventing an imminent violation of the distance criteria between the first and second three-dimensional digital dental model. For preventing such an imminent violation of a distance criterion, a description of an interaction, e.g., a collision of two rigid bodies may be used. When a first rigid body interacts with an obstacle in form of a second rigid body at a point that does not lie on a trajectory of movement of the first rigid body's center of mass, the contact force produces a torque, causing the first rigid body to start or change rotating. More generally, let P be a point on the first rigid body where it contacts the obstacle, i.e., the second rigid body, and let G be the first rigid body's center of mass. Then, a vector r = GP = P - G may be defined. If r is non-zero, any force at P will generally produce a torque about G. For the present interaction, the second rigid body as an obstacle may, e.g., be locked in place, i.e., remain at rest before, during, and after the interaction with the first rigid body. All impulse may be absorbed by the first rigid body, while the obstacle in form of the second rigid body remains unmoved.

For example, the transforming and adjusting may in addition be executed in a symmetric way on a third three-dimensional digital dental model. For example, a third three-dimensional digital dental model of a third dental structure may be received. For example, the third three-dimensional digital dental model may be received together with the first three-dimensional digital dental model. The third three-dimensional digital dental model may be paired with the first three-dimensional digital dental model. For example, the third three-dimensional digital dental model is a contralateral counterpart tooth model to a tooth model provided by the first three-dimensional digital dental model. For example, the third dental structure is a contralateral counterpart tooth to a tooth provided by the first three-dimensional digital dental model. For example, the transforming and adjusting of the first three-dimensional digital dental model is mirrored onto the third three-dimensional digital dental model, i.e., the transforming and adjusting of the first three-dimensional digital dental model is mirror symmetrically mapped onto the third three-dimensional digital dental model.

For example, the mapped transforming and adjusting of the third three-dimensional digital dental model may be a replica of the transforming and adjusting of the first three-dimensional digital dental model relative to a local coordinate system of the third three-dimensional digital dental model. Thus, a rotation of the first three-dimensional digital dental model around a first axis of rotation defined relative to a local coordinate system of the first three-dimensional digital dental model may be mapped to an identical rotation of the third three-dimensional digital dental model around a second axis of rotation defined relative to a local coordinate system of the third three-dimensional digital dental model. The second axis of rotation may be defined relative to the local coordinate system of the third three-dimensional digital dental model in an identical way as the first axis of rotation is defined relative to the local coordinate system of the first three-dimensional digital dental model.

For example, one of the two local coordinate systems may be a right-handed coordinate system, while the other one may be a left-handed coordinate system. This may, e.g., be the case for a pair of corresponding tooth models in form of contralateral counterpart tooth models provided by the first and third three-dimensional digital dental models.

Due to the mapped transforming and adjusting being a replica relative to the local coordinate system of the third three-dimensional digital dental model, the mapping of the transforming and adjusting may be enabled even between tooth models that do not satisfy an exact global symmetry. For example, the first and third three-dimensional digital dental model may not satisfy an exact global symmetry with respect to a mirror plane provided by a sagittal plane dividing an arrangement of three-dimensional digital dental models, e.g., a dental arch, comprising the first and third three-dimensional digital dental model into two halves. For example, the first and third three-dimensional digital dental model may not satisfy an exact global symmetry. For example, the transforming and adjusting applied to the first and third three-dimensional digital dental model may not satisfy an exact global symmetry with respect to the mirror plane provided by the sagittal plane dividing the arrangement of three-dimensional digital dental models, e.g., the dental arch, comprising the first and third three-dimensional digital dental model into two halves.

For example, the first and third three-dimensional digital dental model may satisfy an exact global symmetry with respect to a mirror plane provided by a sagittal plane dividing an arrangement of three-dimensional digital dental models, e.g., a dental arch, comprising the first and third three-dimensional digital dental model into two halves. For example, the first and third three-dimensional digital dental model may satisfy an exact global symmetry. For example, the transforming and adjusting applied to the first and third three-dimensional digital dental model may satisfy an exact global symmetry with respect to the mirror plane provided by the sagittal plane dividing the arrangement of three-dimensional digital dental models, e.g., the dental arch, comprising the first and third three-dimensional digital dental model into two halves.

A local coordinate system of a three-dimensional digital dental model, also referred to as local dental frame herein, may, e.g., be any local coordinate system assigned to a three-dimensional digital dental model. The local coordinate systems assigned to the three-dimensional digital dental models may, e.g., be defined the same way relative to the respective individual three-dimensional digital dental models using anatomical directions of the respective dental models, e.g., tooth models. This may, e.g., result in a switch of handedness for the local coordinate systems of three-dimensional digital dental models of different hemispheres of a jaw or dental arch.

When transforming and adjusting a three-dimensional digital dental model, also the local coordinate system may be transformed and adjusted. For example, when moving and/or rotating a three-dimensional digital dental model, the local coordinate system of the respective three-dimensional digital dental model may be moved and/or rotated as well. Thus, position and/or orientation of the local coordinate systems of different three-dimensional digital dental models may, e.g., differ from each other depending on differences of the position and/or orientation of the respective three-dimensional digital dental models.

The local coordinate system of a three-dimensional digital dental model may, e.g., be defined by anatomical dental directions, i.e., axes. Anatomical dental axes may, e.g., be defined parallel to anatomical directions of the respective dental model, e.g., tooth directions of a tooth model. Anatomical dental axes and/or directions may be defined relative to anatomical sections of a dental model. For example, anatomical sections of a dental model may be defined the same way for all dental models. For example, for each dental model a buccal, a mesial and/or an occlusal anatomical section may be defined the same way. Thus, for each dental model a buccal, a mesial and/or an occlusal axis, i.e., direction may be defined the same way. For example, the same local reference mesh shape defining these directions may be used for each of the dental models. The dental models may, e.g., be library teeth, for which the respective same local reference mesh shape is defined. Even if there is no such pre-defined alignment reference structure, like a mesh shape, anatomical dental axes and/or directions may be determined. For example, in case dental models are provided by scanning natural teeth or physical dental models and segmenting the same, anatomical dental axes and/or directions of the resulting three-dimensional dental models may be determined and local coordinate frames assigned to the dental models.

In reality, teeth of a dentition may, e.g., not be arranged in an arrangement satisfying an exact global symmetry, like a mirroring symmetry. Thus, in order to ensure a realistic and natural appearance of a denture model, dental models, e.g., tooth models, may be arranged in an arrangement not satisfying an exact global symmetry, like a mirroring symmetry. Examples may allow for a mapping of any type of transforming and/or adjusting, like rotation, translation and/or scaling, in one local dental frame of one three-dimensional digital dental model to another local dental frame of another three-dimensional digital dental model. For example, the mapping may be a mapping from the first three-dimensional digital dental model to the third three-dimensional digital dental model descriptive of an opposite contralateral counterpart tooth arranged in a hemisphere of a jaw or dental arch opposite to a hemisphere, in which the first three-dimensional digital dental model is arranged.

Examples may have the beneficial effect that a mapping, e.g., mirroring, may even be possible in case of an arrangement of the three-dimensional digital dental models with no exact global symmetry, e.g., a quasi-symmetrical arrangement. As a result, a performing of any transforming and/or adjusting of a first three-dimensional digital dental model with respect to the local coordinate system, i.e., local dental frame, of the first three-dimensional digital dental model may easily be mirrored to the local coordinate system, i.e., local dental frame, of the paired third three-dimensional digital dental model.

For example, the first three-dimensional digital dental model may be comprised by a set of three-dimensional digital dental models. The set of three-dimensional digital dental models may, e.g., comprise three-dimensional digital dental models of the same dental arch. For example, the set of three-dimensional digital dental models may comprise three-dimensional digital dental models of a full dental arch.

For example, the set of three-dimensional digital dental models may be configured such that it reacts in a chain-like manner to the transforming and adjusting of the first three-dimensional digital dental model. The reaction may, e.g., comprise a transferring of the transforming and adjusting of the first three-dimensional digital dental model to one or more further three-dimensional digital dental models of the set of three-dimensional digital dental models. For example, translational movements and/or rotations of the first three-dimensional digital dental model during the transforming and adjusting of the transforming may be transferred to further three-dimensional digital dental models of the set of three-dimensional digital dental models. For instance, the transforming and adjusting of the first three-dimensional digital dental model may result in a transforming and adjusting of the further three-dimensional digital dental models of the set of three-dimensional digital dental models such that an arrangement of the set of three-dimensional digital dental models on a curved arch is preserved. The curved arch may, e.g., be descriptive of a curved form of a ridge of a jaw. For instance, the transforming and adjusting of the first three-dimensional digital dental model may result in a transforming and adjusting of the further three-dimensional digital dental models of the set of three-dimensional digital dental models such that predefined relative distances between the three-dimensional digital dental models of the set of three-dimensional digital dental models on the curved arch are preserved. For instance, the transforming and adjusting of the first three-dimensional digital dental model may result in a transferring of the transforming and adjusting to further three-dimensional digital dental models of the set of three-dimensional digital dental models with a degree of the transferred transforming and adjusting decreasing with increase of a distance of the respective further three-dimensional digital dental models. Thus, the transforming and adjusting may, e.g., be propagated along the curved arch.

For example, the one or more distance criteria define a threshold for a minimum distance between the first and second three-dimensional digital dental model. This threshold may not be exceeded by the transforming and adjusting, i.e., the transforming and adjusting may be prevented from resulting in a distance between the first and second three-dimensional digital dental model smaller than the threshold.

For example, the threshold for the minimum distance is one of the following: a threshold of size zero; a threshold of positive size; a threshold of negative size. A threshold of size zero refers to a surface contact between the first and second three-dimensional digital dental model. A threshold of positive size defines a distance between the surfaces of the first and second three-dimensional digital dental model. A threshold of negative size defines a penetration of the second three-dimensional digital dental model by the first three-dimensional digital dental model with a penetration depth equaling an absolute value of the threshold.

Examples may have the beneficial effect that an automatic collision prevention may be achievable, as a result of defining a threshold minimum distance of positive size that acts as a buffer to avoid interpenetration between the dental models.

Examples may have the beneficial effect that patient-specific clearance control between the dental models may be achievable, as a result of defining a threshold minimum distance of positive size. This may, e.g., allow for a customization of a gap between the dental models.

Examples may have the beneficial effect that an exact surface contact modeling may be achievable, as a result of using a threshold of size zero to enable a direct contact between the dental models.

Examples may have the beneficial effect that a controlled interference modeling may be achievable, as a result of using a threshold of negative size to intentionally allow for overlapping geometries.

For example, the method may comprise selecting a threshold for the minimum distance. For example, a threshold of size zero, of positive size, or of negative size may be selected.

Examples may have the beneficial effect that a versatile simulation and adjustment of a relation, e.g., an occlusal relation between the dental models is achievable. By selecting the threshold to be of zero, positive, or negative size, an exact contact, a controlled gap, or an intentional overlap may be presented.

Examples may have the beneficial effect that a broad range of clinical fit scenarios between the dental models may be achievable.

For example, the one or more distance criteria define a fixed minimum distance to be enforced between the first and second three-dimensional digital dental model. When enforcing a fixed minimum distance between the first and second three-dimensional digital dental model, the respective minimum distance will be maintained during transforming and adjusting of the first three-dimensional digital dental model. Thus, only those transforming and adjusting measures may be allowed that preserve the fixed minimum distance.

For example, the fixed minimum distance is one of the following: a fixed minimum distance of size zero; a fixed minimum distance of positive size; a fixed minimum distance of negative size.

Examples may have the beneficial effect that patient-specific clearance control between the dental models may be achievable, as a result of defining a fixed minimum distance of positive size. This may, e.g., allow for a customization of a gap between the dental models.

Examples may have the beneficial effect that an exact surface contact modeling may be achievable, as a result of setting a fixed minimum distance of zero to ensure a direct contact between the dental models.

Examples may have the beneficial effect that a controlled interference modeling may be achievable, as a result of assigning a negative fixed minimum distance to intentionally model overlapping geometries.

The one or more distance criteria to be preserved are distance criteria defined between a first surface section of the first three-dimensional digital dental model and a second surface section of the second three-dimensional digital dental model.

By defining a fixed minimum distance of size zero, i.e., a contact between the first and the second three-dimensional digital dental model as a boundary condition to be preserved by the transforming and adjusting, e.g., a sticking between the first and the second three-dimensional digital dental model may be implemented. The sticking may enforce that the first and the second three-dimensional digital dental model remain in contact, at least with one point.

For example, the first and second dental structures described by the first and the second three-dimensional digital dental model may be antagonistic teeth. Thus, a sticking between antagonists may be established. For example, the first and second dental structures described by the first and the second three-dimensional digital dental model may be neighboring teeth. Thus, a sticking between neighboring teeth may be established.

For example, a sticking may also be implemented with a positive or negative offset using a fixed minimum distance of positive size or negative size. For example, positive or negative offset may be an offset between antagonists. For example, positive or negative offset may be an inter-tooth offset.

In case of a negative offset, a resulting intersection between the first and the second three-dimensional digital dental model may, e.g., be resolved. The intersection may, e.g., be resolved by a deforming or a cutting of the first three-dimensional digital dental model. For example, the intersection may be resolved at an end of the adjusting.

For example, the first and second dental structure are antagonistic dental structures. For example, the first surface section of the first three-dimensional digital dental model is an occlusal surface section of the first three-dimensional digital dental model. For example, the second surface section of the second three-dimensional digital dental model is an occlusal surface section of the second three-dimensional digital dental model.

For example, the first and second dental structure are neighboring dental structures. For example, the first surface section of the first three-dimensional digital dental model is a mesial or distal surface section of the first three-dimensional digital dental model. For example, the second surface section of the second three-dimensional digital dental model is a mesial or distal surface section of the second three-dimensional digital dental model.

For example, the method further comprises displaying, during the adjusting of the transforming, a first visual indicator which indicates the start orientation as a first reference orientation using the stored definition of the start orientation.

Examples may have the beneficial effect that a clear orientation reference visualization may be achievable, as a result of displaying a first visual indicator that marks the start orientation using the stored definition.

Examples may have the beneficial effect that a real-time adjustment guidance may be achievable, due to the displaying of the first visual indicator during the adjusting.

Examples may have the beneficial effect that start-state reproducibility may be achievable, as a result of storing the start orientation definition for future reference.

Examples may have the beneficial effect that a seamless return-to-reference operation may be achievable, as a result of the first visual indicator displaying the stored start orientation during the adjusting.

For example, the first visual indicator is displayed upon satisfying a displaying criterion. For example, the displaying criterion is a starting of a decreasing of the angle of rotation around the axis of rotation.

For example, the displaying of the first visual indicator is terminated upon satisfying a termination criterion. For example, the termination criterion is an ending of the adjusting of the transforming. For example, the termination criterion is a detecting of a pressing of an activation button. For example, the termination criterion is a detecting of a releasing of the activation button.

For example, the start orientation is defined by an orientation of a local coordinate frame of the first three-dimensional digital dental model at the start of the rotation. For example, the local coordinate frame is a local Cartesian coordinate system. For example, the local Cartesian coordinate frame at the start of the rotation defines an occlusal, mesial, and buccal direction of the first three-dimensional digital dental model at the start of the rotation.

For example, the provisional end orientation is defined by an orientation of the local coordinate frame of the first three-dimensional digital dental model at the end of the rotation. For example, the local coordinate frame is a local Cartesian coordinate system. For example, the local Cartesian coordinate frame at the end of the rotation defines the occlusal, mesial, and buccal direction of the first three-dimensional digital dental model at the end of the rotation.

For example, the definition of the start orientation is stored upon satisfying a first storing criterion. For example, the first storing criterion is a starting of the positioning. For example, the definition of the start orientation is stored automatically upon starting of the positioning. For example, the first storing criterion is a starting of the rotation. For example, the definition of the start orientation is stored automatically upon starting of the rotation. For example, the first storing criterion is a detecting of a pressing of the activation button.

For example, the method further comprises a deleting of the definition of the start orientation upon satisfying a first deleting criterion. For example, the first deleting criterion is an ending of the adjusting of the transforming. For example, the definition of the start orientation is deleted automatically upon ending of the adjusting of the transforming. For example, the first deleting criterion is a detecting of a pressing of the activation button. For example, the first deleting criterion is a detecting of a releasing of the activation button.

For example, the first visual indicator indicates a direction of the start orientation of the first three-dimensional digital dental model as a first reference direction.

Examples may have the beneficial effect that an intuitive directional context for the adjusting of the transforming may be provided. For this purpose, the first visual indicator may highlight the first reference direction, i.e., the direction of the start orientation of the first three-dimensional digital dental model.

Examples may have the beneficial effect that a faster spatial comprehension may be achievable, as a result of representing the first reference direction, i.e., the direction of the start orientation explicitly.

For example, the first reference direction is an initial occlusal direction of the start orientation of the first three-dimensional digital dental model.

For example, the first visual indicator comprises a first segment pointing in the first reference direction.

Examples may have the beneficial effect that a precise angular reference may be achievable, as a result of the segment providing a clear vector for orientation.

For example, the first segment is a first straight line. For example, the first visual indicator is a first arrow pointing in the first reference direction. The first arrow may, e.g., be a start orientation vector.

For example, the method further comprises displaying, during the adjusting of the transforming, a second visual indicator which indicates the current orientation of the first three-dimensional digital dental model.

Examples may have the beneficial effect that an immediate feedback on orientation may be achievable, as a result of displaying a second visual indicator that shows the current orientation of the first three-dimensional digital dental model during the adjusting. In particular, a relative relation between the start orientation and the current orientation may be indicated by displaying the first and second visual indicators.

Examples may have the beneficial effect that a continuous alignment refinement may be achievable, as a result of the user being able to observe and adjust the current orientation in real time.

For example, the second visual indicator is displayed upon satisfying the displaying criterion. For example, the displaying of the second visual indicator is terminated upon satisfying the termination criterion.

For example, the current orientation of the first three-dimensional digital dental model is defined by an orientation of the local coordinate frame of the first three-dimensional digital dental model at the current angle relative to the start orientation. For example, the local coordinate frame is a local Cartesian coordinate system. For example, the local Cartesian coordinate frame at the current angle relative to the start orientation defines the occlusal, mesial, and buccal direction of the first three-dimensional digital dental model at the current angle.

For example, the second visual indicator indicates a direction of the current orientation of the first three-dimensional digital dental model during the adjusting as a current direction.

Examples may have the beneficial effect that a clear current-direction indication may be achievable, as a result of the second visual indicator revealing the direction of the current orientation of the first three-dimensional digital dental model during the adjusting.

Examples may have the beneficial effect that an accurate deviation assessment may be achievable, as a result of comparing the current direction against the stored reference direction.

For example, the current direction is a current occlusal direction of the current orientation of the first three-dimensional digital dental model during the adjusting.

For example, the second visual indicator comprises a second segment pointing in the current direction.

Examples may have the beneficial effect that a precise angular indication may be achievable, as a result of the segment serving as a precise indicator of the current direction.

For example, the second segment is a second straight line. For example, the second visual indicator is a second arrow pointing in the current direction. The second arrow may, e.g., be a current orientation vector.

For example, the method further comprises storing a definition of the provisional end orientation for further use. During the adjusting of the transforming, a third visual indicator indicating the provisional end orientation as a second reference orientation is displayed using the stored definition of the provisional end orientation.

Examples may have the beneficial effect that end-state reproducibility may be achievable, as a result of storing the provisional end orientation definition for future reference.

Examples may have the beneficial effect that a seamless return-to-reference operation may be achievable, as a result of the third visual indicator displaying the stored provisional end orientation during the adjusting.

For example, the third visual indicator is displayed upon satisfying the displaying criterion. For example, the displaying of the third visual indicator is terminated upon satisfying the termination criterion.

For example, the definition of the provisional end orientation is stored upon satisfying a second storing criterion. For example, the second storing criterion is a starting of a decreasing of the current angle relative to the start orientation.

For example, the method further comprises a deleting of the definition of the provisional end orientation upon satisfying a second deleting criterion. For example, the second deleting criterion is an ending of the adjusting of the transforming. For example, the definition of the provisional end orientation is deleted automatically upon ending of the adjusting of the transforming. For example, the second deleting criterion is a pressing of the activation button. For example, the second deleting criterion is a releasing of the activation button.

For example, the third visual indicator indicates a direction of the provisional end orientation of the first three-dimensional digital dental model as a second reference direction.

Examples may have the beneficial effect that a comparative orientation visualization may be achievable, as a result of indicating the provisional end orientation's direction as a second reference.

Examples may have the beneficial effect that a comprehensive alignment assessment with respect to the start and provisional end orientation may be achievable, as a result of having directional indicators displayed for start, current, and provisional end orientations.

For example, the second reference direction is an occlusal direction of the provisional end orientation of the first three-dimensional digital dental model.

For example, the third visual indicator comprises a third segment pointing in the second reference direction.

Examples may have the beneficial effect that a concise end-reference visualization may be achievable, as a result of using a third segment to denote the second reference direction.

Examples may have the beneficial effect that precise repositioning instructions may be available which are provided by the third segment conveying the second reference direction.

For example, the third segment is a third straight line. For example, the third visual indicator is a third arrow pointing in the second reference direction. The third arrow may, e.g., be a provisional end orientation vector.

For example, the axis of rotation is an axis extending in a mesial direction of the first three-dimensional digital dental model.

For example, an orientation of the axis of rotation is preserved during the rotating of the positioning and/or during the further rotating of the adjusting of the transforming.

For example, the first three-dimensional digital dental model is rotated within a plane extending perpendicular to the axis of rotation, e.g., the mesial direction of the first three-dimensional digital dental model.

For example, the positioning comprises a moving of the first three-dimensional digital dental model from an initial first position to a second position. Thus, a translational movement from the initial first position to the second position may be comprised. For example, the start orientation of the first three-dimensional digital dental model is an orientation of the first three-dimensional digital dental model at the initial first position. For example, the provisional end orientation of the first three-dimensional digital dental model is an orientation of the first three-dimensional digital dental model at the second position.

For example, the adjusting of the transforming comprises an adjusting of the moving of the first three-dimensional digital dental model.

For example, the method further comprises an updating of the definition of the provisional end orientation using a turning orientation of the first three-dimensional digital dental model reached at a turning point during the adjusting of the transforming. The turning point is a point at which the adjusting turns from a temporary increasing of the angle relative to the start orientation to a temporary decreasing. A turning orientation may, e.g., indicate a new provisional end orientation with a smaller angle than the maximum angle reached during positioning. Using an updated end orientation may, e.g., be beneficial when interpolating the current orientation of the first three-dimensional digital dental model during the adjusting between the end and start orientation. A smaller angle between end and start orientation may, e.g., increase an accuracy of the interpolating.

Examples may have the beneficial effect that an adaptive reference updating may be achievable as a result of redefining the provisional end orientation based on a turning point, i.e., on the actual turning orientation reaching the turning point.

Examples may have the beneficial effect that user-intended maximum orientations may be achievable, as a result of capturing the moment of reversal and updating the reference accordingly.

For example, the adjusting of the transforming of the first three-dimensional digital dental model comprises an interpolation of the current orientation of the first three-dimensional digital dental model using the definition of the start orientation and the definition of the provisional end orientation.

Examples may have the beneficial effect that a smooth transitional rotation may be achievable, as a result of interpolating the current orientation between the stored start and end definitions.

Examples may have the beneficial effect that an accurate intermediate orientation may be achievable, as a result of the interpolation algorithm computing precise angles at any adjustment stage.

For example, the interpolation is a spherical interpolation. A spherical interpolation uses a smooth curve on a unit sphere that connects two or more points while staying on the sphere's surface. It may, e.g., be defined as a map γ(t) = Exp_{p₀}(h(t) Log_{p₀}(p₁)) or a multi-point spline analog of this map with p₀ and p₁ being unit-length vectors, Exp_{p₀} being a Riemannian exponential map on the unit sphere, Log_{p₀}(p₁) being an inverse exponential Riemannian map on the unit sphere giving the shortest tangent displacement at p₀ that reaches p₁, h(t) with t∈[0,1]being a monotonically increasing scalar function h:[0,1] → [0,1] with h(0) = 0 and h(1) = 1. By selecting h(t) and additional control points, a linear vs. non-linear timing and a smoothness of the interpolation may be selected.

For example, the interpolation is a spherical linear interpolation. A spherical linear interpolation (SLERP) allows for a smooth interpolating between two points on a surface of a unit sphere. It may, e.g., be used to interpolate between two unit vectors or two unit quaternions, ensuring that interpolated values also lie on the unit sphere and move at a constant angular velocity along a shortest path, i.e., great-circle arc, between them. Any two distinct points on a surface of a unit sphere may define a unique great circle, i.e., the intersection of the sphere with a plane through its center. SLERP moves along this great-circle, tracing a shortest spherical arc between the points. Unlike, e.g., linear interpolation in Euclidean space, SLERP ensures that an interpolation parameter t ∈ [0,1] corresponds to a constant angular step along the arc.

Given two unit vectors or quaternions p₀ and p₁, let cosθ = p₀·p₁ with θ being the angle between them (0 ≤ θ ≤ π). Then for t ∈ [0,1], $SLERP \left({p}^{0}, {p}^{1}, t\right) = \frac{sin \left(\left(1-t\right)θ\right)}{sin θ} {p}_{0} + \frac{sin \left(t θ\right)}{sin θ} {p}_{1} .$

The denominator sin θ normalizes the weights so the result stays on the unit sphere. The weights sin((1-t) θ) ensure the path follows the great-circle and moves at constant angular rate. This may, e.g., also be used to smoothly interpolate unit quaternions representing three-dimensional orientations. Examples may ensure a shortest-path rotation, i.e., the interpolation may always use a rotation by a minimal angle. Furthermore, a constant angular velocity may be ensured.

In spherical interpolation of orientations, each orientation may be treated as a point on the 4D unit sphere via its quaternion. A quaternion q has one real part w and three imaginary parts (x, y, z). Spherical linear interpolation smoothly rotates from one quaternion to another along the hyperspherical geodesic.

When spherical linear interpolation is applied to unit quaternions, a quaternion path maps to a path through three-dimensional rotations in a standard way. The effect is a rotation with uniform angular velocity around a fixed rotation axis. When the initial end point is the identity quaternion, the spherical linear interpolation gives a segment of a one-parameter subgroup of both the Lie group of three-dimensional rotations, SO(3), and its universal covering group of unit quaternions, S³. A spherical linear interpolation may give a straightest and shortest path between its quaternion end points, and maps to a rotation through an angle of 2θ.

Compared to a linear interpolation, a spherical linear interpolation may have the advantage of using a great-circle on a unit sphere rather than a straight line, which may allow for a uniform angular speed and integrated constraint preservation by naturally staying on the unit sphere.

Executing the spherical linear interpolation may, e.g., comprise calculating the interpolation parameter t between a start and end orientation for determining the current position. The interpolation parameter t may be a value between 0 and 1. The parameter t is, e.g., used for the spherical linear interpolation to interpolate an orientation of the current reference coordinate frame between the start and end reference coordinate frames for the respective parameter t between 0 and 1.

The spherical linear interpolation may, e.g., be used for determining an updated interpolated current reference coordinate frame. The determining of the updated interpolated current reference coordinate frame may, e.g., comprise determining a rotation matrix for rotating the current reference coordinate frame with its current orientation to an updated interpolated current orientation. For determining the rotation matrix, a current position of the first three-dimensional digital dental model may be used for providing a pivot for the respective rotation. The rotation matrix may, e.g., be used to back or forward correct, i.e., adjust, an orientation of the first three-dimensional digital dental model. For example, reference directions indicating the orientations of all three reference coordinate frames, i.e., start, end, and current reference coordinate frame, may be visualized.

For example, the current orientation of the first three-dimensional digital dental model may be interpolated using the definition of the start orientation and the definition of the provisional end orientation for a back rotation and/or for a forward rotation of the first three-dimensional digital dental model comprised by the adjusting of the transforming.

For example, the transforming of the first three-dimensional digital dental model comprises a scaling of the first three-dimensional digital dental model. For example, the adjusting of the transforming of the first three-dimensional digital dental model comprises an adjusting of the scaling of the first three-dimensional digital dental model. For example, a definition of a start size of the first three-dimensional digital dental model is stored at a start of the scaling. For example, the definition of the start size is stored together with the definition of the start orientation. For example, a definition of an end size is stored at an end of the scaling. For example, the definition of the end size is stored together with the definition of the provisional end orientation. For example, the adjusting of the scaling comprises an interpolation of a current size of the first three-dimensional digital dental model using the definition of the start size and the definition of the end size.

In case a stick-in-contact feature is active, i.e., a fixed minimum distance between the first and second three-dimensional digital dental model is enforced, a closest point of the first three-dimensional digital dental model relative to the second three-dimensional digital dental model may be determined and a relative distance between the first and second three-dimensional digital dental model at the determined point adjusted such that the relative distance corresponds to the minimum distance.

For example, an imminent violation of the distance criteria between the first and second three-dimensional digital dental model may, e.g., be prevented using a description of an interaction, e.g., in form of a collision of two rigid bodies. Thus, the distance criteria may, e.g., be enforced by defining the first and second three-dimensional digital dental model as two rigid bodies. The interaction between these two rigid bodies may, e.g., be defined such that a minimum distance between the first and second three-dimensional digital dental model cannot be decreased below a predefined threshold. The respective threshold may, e.g., be one of the following: a threshold of size zero; a threshold of positive size; a threshold of negative size. In case the respective threshold is reached, the interaction between the first and second three-dimensional digital dental model may, e.g., be described by a collision interaction. For a threshold of size zero, surfaces of the rigid bodies may, e.g., be defined by the surfaces of the first and second three-dimensional digital dental model. For a threshold of positive size, the surface of the first rigid body may, e.g., be defined by a positive offset of the surfaces of the first three-dimensional digital dental model and/or the surface of the second rigid body may, e.g., be defined by a positive offset of the surface of the second three-dimensional digital dental model. For a threshold of negative size, the surface of the first rigid body may, e.g., be defined by a negative offset of the surfaces of the first three-dimensional digital dental model and/or the surface of the second rigid body may, e.g., be defined by a negative offset of the surface of the second three-dimensional digital dental model.

When the predefined threshold is reached, the first three-dimensional digital dental model may, e.g., interact as a first rigid body with the second three-dimensional digital dental model forming an obstacle for the first rigid body. The interaction may, e.g., cause a contact force between the two rigid bodies producing a torque for the first rigid body. The torque may, e.g., cause the first rigid body to start or change rotating. For the present interaction, the second rigid body as an obstacle may, e.g., be locked in place, i.e., remain at rest before, during, and after the interaction with the first rigid body. All impulse may thus be absorbed by the first rigid body, while the obstacle in form of the second rigid body may remain unmoved.

Such a collision interaction may, e.g., induce a rotation of the first three-dimensional digital dental model. For example, such a collision interaction may further induce a translational movement of the first three-dimensional digital dental model.

For example, the induced rotation may be constrained to a mesial plane of the first three-dimensional digital dental model, i.e., a plane which has the mesial axis of the local coordinate frame of the first three-dimensional digital dental model as normal. For example, the induced rotation may not be constrained to a particular plane of the first three-dimensional digital dental model.

For example, in case an imminent violation of the distance criteria between the first and second three-dimensional digital dental model is to be prevented, the provisional end orientation of the three-dimensional digital dental model may, e.g., be updated using a current orientation of the three-dimensional digital dental model. For example, the stored provisional end orientation of the three-dimensional digital dental model may be updated, i.e., replaced by the current orientation of the three-dimensional digital dental model. Examples may have the beneficial effect that for a back-correction, i.e., back-rotation, towards the start orientation taking a latest orientation as the provisional end orientation may improve a precision of an interpolation compared to using the provisional end orientation reached at the maximum angle during positioning.

For example, the provisional end orientation may be maintained during the adjusting without updating the same.

For example, when adjusting the transforming of the first three-dimensional digital dental model, definitions of the start orientation, the provisional end orientation, and the current orientation of the first three-dimensional digital dental model may be provided. The start orientation and provisional end orientation may be provided by stored definitions.

For example, the definition of the start orientation may comprise a start reference coordinate frame defining the start orientation of the first three-dimensional digital dental model. For example, the definition of the provisional end orientation may comprise an end reference coordinate frame defining the provisional end orientation of the first three-dimensional digital dental model. For example, the definition of the current orientation may comprise a current reference coordinate frame defining the current orientation of the first three-dimensional digital dental model. The individual reference coordinate frames may, e.g., be defined by local coordinate systems of the first three-dimensional digital dental model arranged with the respective orientation. The coordinate systems may, e.g., be Cartesian coordinate systems. Coordinate axes of the coordinate systems may, e.g., be defined by anatomical directions, like, e.g., occlusal, mesial, and/or buccal direction.

For example, the start orientation of the first three-dimensional digital dental model may, e.g., be reset, i.e., updated, upon one or more of the following: a pressing of an activation button, a reaching of the threshold for the minimum distance between the first and second three-dimensional digital dental model, a reaching of the start position.

For example, the first dental structure is a first tooth. For example, the second dental structure comprises a second tooth. For example, the second dental structure is the second tooth.

For example, the second dental structure comprises one or more antagonistic dental structures antagonistic to the first dental structure. For example, the antagonistic dental structure comprises one or more antagonist teeth. For example, the antagonistic dental structure is the antagonist tooth. For example, the second dental structure comprises one or more neighboring dental structures neighboring the first dental structure. For example, the neighboring dental structure comprises one or more antagonist teeth. For example, the neighboring dental structure is the neighboring tooth.

For example, the second three-dimensional digital dental model may further comprise one or more of the following: a three-dimensional digital neighboring dental model; a three-dimensional digital teeth model comprising a set of teeth, within which the first three-dimensional digital dental model is arranged; a three-dimensional digital antagonistic tooth model; a three-dimensional digital antagonist teeth model comprising a set of three-dimensional digital tooth models with a three-dimensional digital antagonistic tooth model; a three-dimensional digital dental arch model, within which the first three-dimensional digital dental model is arranged; a three-dimensional digital antagonist dental arch model comprising the three-dimensional digital antagonistic tooth model.

For example, the three-dimensional digital dental models may, e.g., be dental structures, like teeth, provided by a library of dental structures, like a tooth library. The three-dimensional digital dental models may, e.g., be provided using scan data of the dental structures. The scan data of the dental structures may, e.g., comprise optical scan data. The optical scan data may, e.g., comprise intraoral optical scan data or optical scan data from an optical scan of a classical mold/impression of the dental structures. The optical scan data may, e.g., provide information about the surface structure of the respective dental structures. The three-dimensional digital dental models may, e.g., be generated from scratch.

For example, the method further comprises generating a three-dimensional digital prosthesis model of a dental prosthesis comprising the adjusted first three-dimensional digital dental model and providing control data for controlling a manufacturing of the dental prosthesis using a manufacturing device. The control data defines the three-dimensional digital prosthesis model.

Examples may have the beneficial effect that an integrated prosthesis design may be achievable, as a result of generating the three-dimensional digital prosthesis model directly from the adjusted dental model. Examples may have the beneficial effect that a direct manufacturing readiness may be achievable.

For example, the method further comprises controlling the manufacturing device to manufacture the dental prosthesis using the control data. For example, the dental prosthesis is manufactured as a physical copy of the three-dimensional digital prosthesis model.

The dental prosthesis may, e.g., be a denture. For example, the dental prosthesis may, e.g., be a partial or a complete denture. A denture is a prosthetic device constructed to replace missing teeth and to be supported by surrounding soft and/or hard tissues of the oral cavity. The denture may, e.g., be a removable denture, e.g., a removable partial denture or a removable complete denture. Alternatively, the denture may, e.g., be a denture relying on bonding or clasping onto teeth or dental implants.

For example, the dental prosthesis may comprise one or more of the following: a veneer, a coping with coating, a crown, a bridge, a mockup, a waxup, a provisional.

A veneer is a layer of restoration material placed over a tooth, in order to cover one or more surfaces of the tooth. Veneers may, e.g., improve the aesthetics of a smile and/or protect the tooth's surface from damage. Indirect veneers are manufactured outside of a patient's oral cavity and then arranged on a tooth within the oral cavity. Direct veneers are built-up directly on a tooth inside a patient's oral cavity. The tooth may be prepared for receiving the veneer.

For example, two main types of restoration material may be used for manufacturing a veneer: composite and dental porcelain. A composite veneer may be directly placed on the tooth, i.e., built-up in the mouth of a patient, or indirectly manufactured outside the mouth of the patient and later bonded to the tooth. In contrast, a porcelain veneer may only be indirectly manufactured outside the mouth of the patient. A full veneer crown, on the one hand, is a dental prosthesis element that is configured to cover all the coronal tooth surfaces, i.e., the mesial, distal, facial, lingual, and occlusal surfaces. A laminate veneer, on the other hand, is a thin layer of restoration material that may, e.g., cover only a single surface of a tooth, e.g., a labial surface. A laminate veneer may generally be used for aesthetic purposes.

Coping with coating refers to a dental prosthesis element, which is directly built on the tooth to be restored. An underlying coping is arranged on the tooth. The coping is configured to replicate the performance of a natural tooth. On the coping a coating is applied, which is configured to replicate the natural aesthetics of the tooth to be restored. For example, a ceramic coating may be used. Using a coping with coating to restore a tooth may have the beneficial effect of providing a dental prosthesis element that combines both durability and natural aesthetics.

A crown is a dental prosthesis element in form of a dental cap. Such a crown may, e.g., be provided in form of a full coverage crown or a partial crown, like a 7/8 crown or a 3/4 crown. Partial crowns, like 7/8 and 3/4 crowns, are hybrids between an onlay and a full coverage crown. They are categorized based on an estimated degree of wall coverage of the walls of the prepared tooth, on which the respective crown is arranged. For example, a 3/4 crown aims to cover three thirds of the walls of the tooth to be restored, e.g., three out of the four walls, e.g., with the buccal wall being spared. For example, a 7/8 crown aims to cover seven eighths of the walls of the tooth to be restored. A full coverage crown completely caps or encircles a prepared tooth. A crown may, e.g., be required when a large cavity threatens the health of a tooth. A crown may be bonded to the tooth prepared for receiving the crown using a bonding material, e.g., a dental cement. A crown may be made from various materials, which may be fabricated using indirect methods, i.e., outside the patient's oral cavity. Crowns may be used to improve strength, to improve appearance of teeth and/or to halt deterioration.

A bridge is a dental prosthesis in form of a permanent appliance used to replace one or more missing teeth. A dental bridge comprises a plurality of artificial dental elements that are fused together, e.g., one or more artificial replacement teeth are definitively joined to adjacent teeth. A conventional bridge may be supported, e.g., by full coverage crowns, partial crowns, overlays, onlays or inlays on the abutment teeth. The abutment teeth require preparation and reduction to support the bridge.

A mockup prosthetic restoration composite is a composite to be arranged within a patient's mouth in order to visualize for the patient a result of a prosthetic restoration, before the actual prosthetic restoration is executed. Thus, the patient as well as a dentist may assess the expected esthetic and functional outcome of the prosthetic restoration. The final result to be expected may thus be visualized at an early stage of planning a prosthetic restoration. This approach may ensure that the patient as well as the dentist may have the same result to be achieved in mind and allows for potential adjustments to be made prior to the final restorations being manufactured and applied, e.g., cemented.

A waxup prosthetic restoration refers to a prosthetic restoration made from laboratory wax. Such a waxup prosthetic restoration is used for acquiring information indicative of whether a specific prosthetic restoration is appropriate. A planned prosthetic restoration may be generated using laboratory wax. The waxup prosthetic restoration may be used to test whether the planned prosthetic restoration is appropriate. Using wax may have the beneficial effect that the waxup prosthetic restoration may be easily adjusted to also test adjustments of the planned prosthetic restoration and/or adjusting the planned prosthetic restoration to requirements determined using the waxup prosthetic restoration.

A waxup model may, e.g., be used by a doctor and/or a practitioner for visualization purposes. Furthermore, it may, e.g., also be used for generating one or more in-mouth preparation guiding surfaces, e.g., using silicone imprints, where the doctor and/or practitioner may measure and/or visually gauge whether a planned tooth reduction has been performed.

A provisional is a type of interim dental prosthesis designed to be a template for the final restoration. It is used to verify, e.g., a comfort in occlusion for the patient, esthetic parameters that satisfy the patient's and dentist's expected goals and/or phonetic evaluation for speech and airflow. Esthetic parameters may, e.g., comprise shape, midlines, smile line, embrasure shapes, and/or position of contacts. The phonetic evaluation for speech and airflow may ensure that no sibilance, whistlers, and/or lisp occur, and a clear articulation is enabled by the prosthetic restoration resembled by the provisional.

For example, a computer program is provided for adjusting a transformation of a first three-dimensional digital dental model of a first dental structure relative to a second three-dimensional digital dental model of a second dental structure.

The computer program comprises program instructions which are executable by a processing unit of a computer device to cause the computer device to receive the first three-dimensional digital dental model. The second three-dimensional digital dental model is received. The first three-dimensional digital dental model is transformed. The transforming comprises a positioning of the first three-dimensional digital dental model relative to the second three-dimensional digital dental model. The positioning of the first three-dimensional digital dental model relative to the second three-dimensional digital dental model is restricted by a boundary condition defining one or more distance criteria to be preserved between the first and second three-dimensional digital dental model. A preserving of the one or more distance criteria by the positioning results in a rotating of the first three-dimensional digital dental model around an axis of rotation increasing an angle of a current orientation of the first three-dimensional digital dental model relative to an initial start orientation of the first three-dimensional digital dental model at a start of the rotation until the current orientation reaches a provisional end orientation of the first three-dimensional digital dental model at an end of the rotation with a maximum angle relative to the start orientation. A definition of the start orientation is stored for further use.

The program instructions are further executable by the processing unit of the computer device to cause the computer device to adjust the transforming of the first three-dimensional digital dental model, in particular the positioning of the first three-dimensional digital dental model. A preserving of the one or more distance criteria by the adjusting of the transforming results in a further rotating of the first three-dimensional digital dental model around the axis of rotation using the stored definition of the start orientation as a first reference orientation. The further rotating at least partially reverses the rotating of the first three-dimensional digital dental model such that the current angle relative to the start orientation is decreased.

The execution of the program instructions by a processing unit of a computer device may, e.g., cause the computer device to execute a method according to any of the examples of a method disclosed herein for adjusting a transformation of a first three-dimensional digital dental model of a first dental structure relative to a second three-dimensional digital dental model of a second dental structure.

For example, a computer program product is provided for adjusting a transformation of a first three-dimensional digital dental model of a first dental structure relative to a second three-dimensional digital dental model of a second dental structure.

The computer program product comprises a non-transitory computer-readable storage medium having program instructions embodied therewith. The program instructions are executable by a processing unit of a computer device to cause the computer device to receive the first three-dimensional digital dental model. The second three-dimensional digital dental model is received. The first three-dimensional digital dental model is transformed. The transforming comprises a positioning of the first three-dimensional digital dental model relative to the second three-dimensional digital dental model. The positioning of the first three-dimensional digital dental model relative to the second three-dimensional digital dental model is restricted by a boundary condition defining one or more distance criteria to be preserved between the first and second three-dimensional digital dental model. A preserving of the one or more distance criteria by the positioning results in a rotating of the first three-dimensional digital dental model around an axis of rotation increasing an angle of a current orientation of the first three-dimensional digital dental model relative to an initial start orientation of the first three-dimensional digital dental model at a start of the rotation until the current orientation reaches a provisional end orientation of the first three-dimensional digital dental model at an end of the rotation with a maximum angle relative to the start orientation. A definition of the start orientation is stored for further use.

The program instructions are further executable by the processing unit of the computer device to cause the computer device to adjust the transforming of the first three-dimensional digital dental model, in particular the positioning of the first three-dimensional digital dental model. A preserving of the one or more distance criteria by the adjusting of the transforming results in a further rotating of the first three-dimensional digital dental model around the axis of rotation using the stored definition of the start orientation as a first reference orientation. The further rotating at least partially reverses the rotating of the first three-dimensional digital dental model such that the current angle relative to the start orientation is decreased.

The execution of the program instructions by a processing unit of a computer device may, e.g., cause the computer device to execute a method according to any of the examples of a method disclosed herein for adjusting a transformation of a first three-dimensional digital dental model of a first dental structure relative to a second three-dimensional digital dental model of a second dental structure.

For example, a computer device is provided for adjusting a transformation of a first three-dimensional digital dental model of a first dental structure relative to a second three-dimensional digital dental model of a second dental structure. The computer device comprises a processing unit and a memory unit storing program instructions executable by the processing unit. An execution of the program instructions by the processing unit causes the computer device to receive the first three-dimensional digital dental model. The second three-dimensional digital dental model is received. The first three-dimensional digital dental model is transformed. The transforming comprises a positioning of the first three-dimensional digital dental model relative to the second three-dimensional digital dental model. The positioning of the first three-dimensional digital dental model relative to the second three-dimensional digital dental model is restricted by a boundary condition defining one or more distance criteria to be preserved between the first and second three-dimensional digital dental model. A preserving of the one or more distance criteria by the positioning results in a rotating of the first three-dimensional digital dental model around an axis of rotation increasing an angle of a current orientation of the first three-dimensional digital dental model relative to an initial start orientation of the first three-dimensional digital dental model at a start of the rotation until the current orientation reaches a provisional end orientation of the first three-dimensional digital dental model at an end of the rotation with a maximum angle relative to the start orientation. A definition of the start orientation is stored for further use.

The program instructions are further executable by the processing unit of the computer device to cause the computer device to adjust the transforming of the first three-dimensional digital dental model, in particular the positioning of the first three-dimensional digital dental model. A preserving of the one or more distance criteria by the adjusting of the transforming results in a further rotating of the first three-dimensional digital dental model around the axis of rotation using the stored definition of the start orientation as a first reference orientation. The further rotating at least partially reverses the rotating of the first three-dimensional digital dental model such that the current angle relative to the start orientation is decreased.

The execution of the program instructions by the processing unit of the computer device may, e.g., cause the computer device to execute a method according to any of the examples of a method disclosed herein for adjusting a transformation of a first three-dimensional digital dental model of a first dental structure relative to a second three-dimensional digital dental model of a second dental structure.

The computer device may, e.g., be a classical computer device and/or a quantum computing device. For example, the computer device may, e.g., comprise a classical processing unit and/or a quantum processing unit. The processing unit comprised by the computer device may, e.g., be a classical processing unit and/or a quantum processing unit.

For example, a manufacturing system is provided which comprises a computer device according to any of the examples of a computer device disclosed herein. The manufacturing system further comprises a manufacturing device configured for manufacturing a dental prosthesis.

An execution of the program instructions by the processing unit further causes the computer device to generate a three-dimensional digital prosthesis model of the dental prosthesis comprising the adjusted first three-dimensional digital dental model. Control data for controlling the manufacturing of the dental prosthesis are provided using the manufacturing device. The control data defines the three-dimensional digital prosthesis model. The manufacturing device is controlled to manufacture the dental prosthesis using the control data. The dental prosthesis is manufactured as a physical copy of the three-dimensional digital prosthesis model.

The control data may, e.g., define a three-dimensional digital prosthesis model according to any of the examples of a three-dimensional digital prosthesis model disclosed herein. The manufacturing device may, e.g., be controlled, using the control data, to manufacture any of the examples of a three-dimensional digital prosthesis model disclosed herein.

Examples may have the beneficial effect that an end-to-end automation may be achievable, as a result of combining the adjustment, prosthesis modeling, and manufacturing control in a single integrated system.

In the following, similar elements are denoted by the same reference numerals.

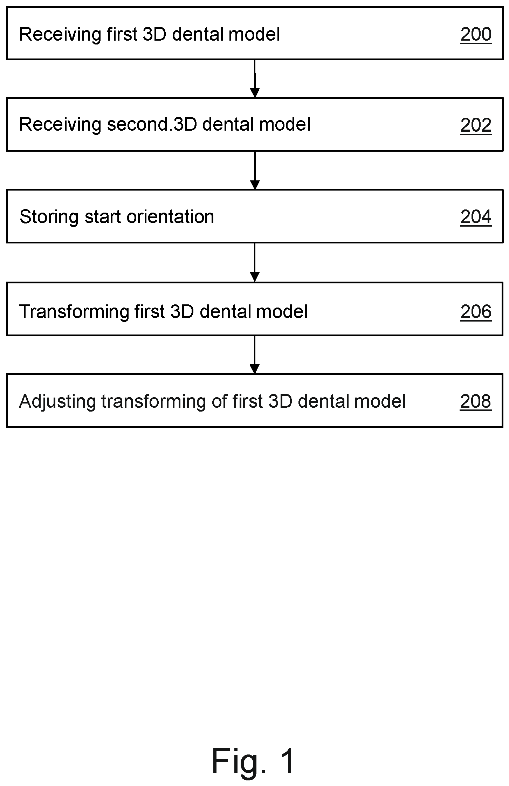

Fig. 1 shows an exemplary computer-implemented method for adjusting a transformation of a first three-dimensional digital dental model of a first dental structure relative to a second three-dimensional digital dental model of a second dental structure. In block 200, the first three-dimensional digital dental model is received. In block 202, the second three-dimensional digital dental model is received.

For example, the first dental structure is a first tooth. For example, the second dental structure comprises a second tooth and/or the second dental structure is the second tooth. For example, the second dental structure comprises one or more antagonistic dental structures antagonistic to the first dental structure. For example, the antagonistic dental structure comprises one or more antagonist teeth and/or the antagonistic dental structure is the antagonist tooth. For example, the second dental structure comprises one or more neighboring dental structures neighboring the first dental structure. For example, the neighboring dental structure comprises one or more antagonist teeth and/or the neighboring dental structure is the neighboring tooth.

For example, the second three-dimensional digital dental model may further comprise one or more of the following: a three-dimensional digital neighboring dental model; a three-dimensional digital teeth model comprising a set of teeth, within which the first three-dimensional digital dental model is arranged; a three-dimensional digital antagonistic tooth model; a three-dimensional digital antagonist teeth model comprising a set of three-dimensional digital tooth models with a three-dimensional digital antagonistic tooth model; a three-dimensional digital dental arch model, within which the first three-dimensional digital dental model is arranged; a three-dimensional digital antagonist dental arch model comprising the three-dimensional digital antagonistic tooth model.

In block 206, the first three-dimensional digital dental model is transformed. The transforming comprises a positioning of the first three-dimensional digital dental model relative to the second three-dimensional digital dental model. The positioning of the first three-dimensional digital dental model relative to the second three-dimensional digital dental model is restricted by a boundary condition which defines one or more distance criteria to be preserved between the first and second three-dimensional digital dental model.

For example, the one or more distance criteria to be preserved are distance criteria defined between a first surface section of the first three-dimensional digital dental model, e.g., a first occlusal section, and a second surface section of the second three-dimensional digital dental model, e.g., a second occlusal section.

For example, the one or more distance criteria may define a threshold for a minimum distance between the first and second three-dimensional digital dental model. This threshold for the minimum distance may, e.g., be a threshold of size zero, a threshold of positive size, or a threshold of negative size. For example, the one or more distance criteria may define a fixed minimum distance to be enforced between the first and second three-dimensional digital dental model. This fixed minimum distance may, e.g., be a fixed minimum distance of size zero, a fixed minimum distance of positive size, or a fixed minimum distance of negative size.

A preserving of the one or more distance criteria defined by the boundary condition during the positioning may result in a rotating of the first three-dimensional digital dental model around an axis of rotation. For example, the axis of rotation is an axis extending in a mesial direction of the first three-dimensional digital dental model. For example, the first three-dimensional digital dental model is rotated within a plane extending perpendicular to the axis of rotation, e.g., the mesial direction of the first three-dimensional digital dental model. When rotating around the axis of rotation, an angle of a current orientation of the first three-dimensional digital dental model relative to an initial start orientation of the first three-dimensional digital dental model at a start of the rotation may be increased until the current orientation reaches a provisional end orientation. The provisional end orientation of the first three-dimensional digital dental model is an orientation at an end of the rotation with a maximum angle relative to the start orientation. For example, an orientation of the axis of rotation is preserved during the rotating of the positioning.

For example, the positioning comprises a moving of the first three-dimensional digital dental model from an initial first position to a second position. For example, the start orientation of the first three-dimensional digital dental model is an orientation of the first three-dimensional digital dental model at the initial first position. For example, the provisional end orientation of the first three-dimensional digital dental model is an orientation of the first three-dimensional digital dental model at the second position.

In block 204, a definition of the start orientation is stored for further use. If the positioning in block 206 starts with the rotating of the first three-dimensional digital dental model, the storing of the definition in block 204 may be executed at the beginning of the positioning of block 206. If the rotating of the first three-dimensional digital dental model starts later during the positioning in block 206, the storing of the definition in block 204 may be executed later during the positioning in block 206. For example, the definition of the start orientation is stored upon satisfying a first storing criterion. For example, the first storing criterion is a starting of the positioning. For example, the definition of the start orientation is stored automatically upon starting of the positioning. For example, the first storing criterion is a starting of the rotation. For example, the definition of the start orientation is stored automatically upon starting of the rotation. For example, the first storing criterion is a detecting of a pressing of an activation button.

For example, the start orientation is defined by an orientation of a local coordinate frame of the first three-dimensional digital dental model at the start of the rotation. For example, the local coordinate frame is a local Cartesian coordinate system. For example, the local Cartesian coordinate frame at the start of the rotation defines an occlusal, mesial, and buccal direction of the first three-dimensional digital dental model at the start of the rotation.

In block 208, the transforming of the first three-dimensional digital dental model is adjusted. A preserving of the boundary condition, i.e., the one or more distance criteria, by the adjusting of the transforming comprises a further rotating of the first three-dimensional digital dental model around the axis of rotation using the definition of the start orientation stored in block 204 as a first reference orientation. The further rotating at least partially reverses the previous rotating of the first three-dimensional digital dental model in block 206 such that the current angle relative to the start orientation is decreased. For example, an orientation of the axis of rotation is preserved during the further rotating of the adjusting of the transforming.

For example, the provisional end orientation is defined by an orientation of the local coordinate frame of the first three-dimensional digital dental model at the end of the rotation. For example, the local coordinate frame is a local Cartesian coordinate system. For example, the local Cartesian coordinate frame at the end of the rotation defines the occlusal, mesial, and buccal direction of the first three-dimensional digital dental model at the end of the rotation.

For example, the further rotating during the adjusting of the transforming comprises a back rotation. For example, the back rotation results in a back correction, which at least partly reverses the positioning. For example, the further rotating during the adjusting of the transforming in addition comprises a forward rotation. For example, the forward rotation results in a forward correction, which at least partly restores the positioning.

For example, the method further comprises displaying, during the adjusting of the transforming in block 208, a first visual indicator indicating the start orientation as a first reference orientation using the definition of the start orientation stored in block 204. For example, the first visual indicator indicates a direction of the start orientation of the first three-dimensional digital dental model as a first reference direction. For example, the first reference direction is an initial occlusal direction of the start orientation of the first three-dimensional digital dental model. For example, the first visual indicator comprises a first segment pointing in the first reference direction. For example, the first segment is a first straight line. For example, the first visual indicator is a first arrow pointing in the first reference direction.

For example, the first visual indicator is displayed upon satisfying a displaying criterion. For example, the displaying criterion is a starting of a decreasing of the angle of rotation around the axis of rotation. For example, the displaying of the first visual indicator is terminated upon satisfying a termination criterion. For example, the termination criterion is an ending of the adjusting of the transforming. For example, the termination criterion is a detecting of a pressing of an activation button. For example, the termination criterion is a detecting of a releasing of the activation button.

For example, the method further comprises displaying, during the adjusting of the transforming in block 208, a second visual indicator indicating the current orientation of the first three-dimensional digital dental model. For example, the second visual indicator is displayed upon satisfying the aforementioned displaying criterion. For example, the displaying of the second visual indicator is terminated upon satisfying the aforementioned termination criterion.

For example, the current orientation of the first three-dimensional digital dental model is defined by an orientation of the local coordinate frame of the first three-dimensional digital dental model at the current angle relative to the start orientation. For example, the local coordinate frame is a local Cartesian coordinate system. For example, the local Cartesian coordinate frame at the current angle relative to the start orientation defines the occlusal, mesial, and buccal direction of the first three-dimensional digital dental model at the current angle.

For example, the second visual indicator indicates a direction of the current orientation of the first three-dimensional digital dental model during the adjusting as a current direction. For example, the current direction is a current occlusal direction of the current orientation of the first three-dimensional digital dental model during the adjusting. For example, the second visual indicator comprises a second segment pointing in the current direction. For example, the second segment is a second straight line. For example, the second visual indicator is a second arrow pointing in the current direction.

For example, the method further comprises storing a definition of the provisional end orientation for further use at the end of the positioning of block 206. For example, the definition of the provisional end orientation is stored upon satisfying a second storing criterion. For example, the second storing criterion is a starting of a decreasing of the current angle relative to the start orientation.

During the adjusting of the transforming in block 208, a third visual indicator indicating the provisional end orientation as a second reference orientation may, e.g., be displayed using the definition of the provisional end orientation stored at the end of the positioning of block 206. For example, the third visual indicator is displayed upon satisfying the aforementioned displaying criterion. For example, the displaying of the third visual indicator is terminated upon satisfying the aforementioned termination criterion.

For example, the third visual indicator indicates a direction of the provisional end orientation of the first three-dimensional digital dental model as a second reference direction. For example, the second reference direction is an occlusal direction of the provisional end orientation of the first three-dimensional digital dental model. For example, the third visual indicator comprises a third segment pointing in the second reference direction. For example, the third segment is a third straight line. For example, the third visual indicator is a third arrow pointing in the second reference direction.

For example, the adjusting of the transforming of the first three-dimensional digital dental model in block 208 comprises an interpolation of the current orientation of the first three-dimensional digital dental model using the definition of the start orientation and the definition of the provisional end orientation. For example, the interpolation is a spherical interpolation, e.g., a spherical linear interpolation.

For example, the method further comprises an updating of the definition of the provisional end orientation during the adjusting of block 208. For the updating, a turning orientation of the first three-dimensional digital dental model reached at a turning point during the adjusting of the transforming may be used. The turning point is a point at which the adjusting turns from a temporary increasing of the angle relative to the start orientation to a temporary decreasing. When a reaching of a turning point is detected, the stored provisional end orientation may be updated, i.e., replaced by the turning orientation of the first three-dimensional digital dental model reached at a turning point.

For example, the adjusting of the transforming in block 208 further comprises an adjusting of a moving of the first three-dimensional digital dental model during the positioning of block 206.

For example, the method further comprises a deleting of the definition of the start orientation upon satisfying a first deleting criterion. For example, the first deleting criterion is an ending of the adjusting of the transforming. For example, the definition of the start orientation is deleted automatically upon ending of the adjusting of the transforming. For example, the first deleting criterion is a detecting of a pressing of the activation button. For example, the first deleting criterion is a detecting of a releasing of the activation button.

For example, the method further comprises a deleting of the definition of the provisional end orientation upon satisfying a second deleting criterion. For example, the second deleting criterion is an ending of the adjusting of the transforming. For example, the definition of the provisional end orientation is deleted automatically upon ending of the adjusting of the transforming. For example, the second deleting criterion is a pressing of the activation button. For example, the second deleting criterion is a releasing of the activation button.

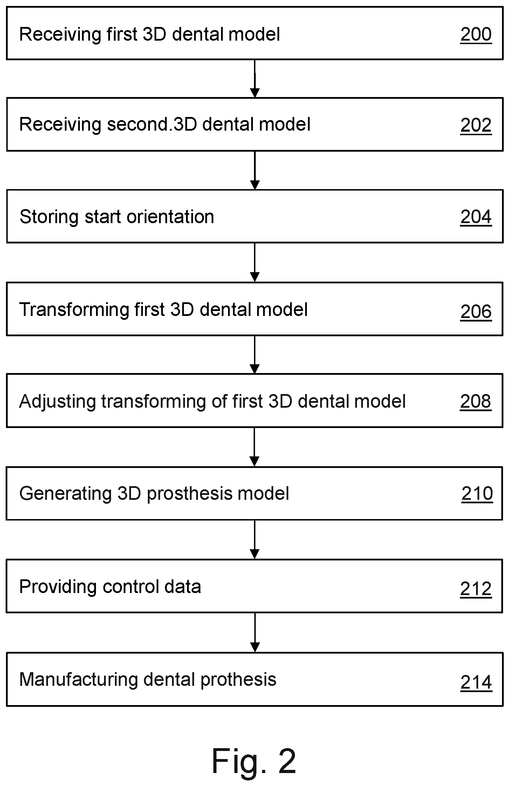

Fig. 2 shows an exemplary computer-implemented method for manufacturing a dental prosthesis. The method may comprise transforming and adjusting the transforming of a first three-dimensional digital dental model of a first dental structure. The transforming may comprise positioning and the adjusting of the transforming may comprise adjusting the positioning of the first three-dimensional digital dental model of the first dental structure relative to a second three-dimensional digital dental model of a second dental structure. The positioning and the adjusting of the transforming may, e.g., comprise blocks 200 to 208 of Fig. 1. The result of block 208 may be the adjusted first three-dimensional digital dental model, i.e., the first three-dimensional digital dental model arranged at an adjusted position relative to the second three-dimensional digital dental model.

In block 210, a three-dimensional digital prosthesis model of the dental prosthesis comprising the adjusted first three-dimensional digital dental model may be generated. In block 212, control data for controlling a manufacturing of the dental prosthesis using a manufacturing device may be provided. The control data may define the three-dimensional digital prosthesis model. In block 214, the manufacturing device may be controlled to manufacture the dental prosthesis using the control data provided in block 212. The dental prosthesis may be manufactured as a physical copy of the three-dimensional digital prosthesis model generated in block 210.

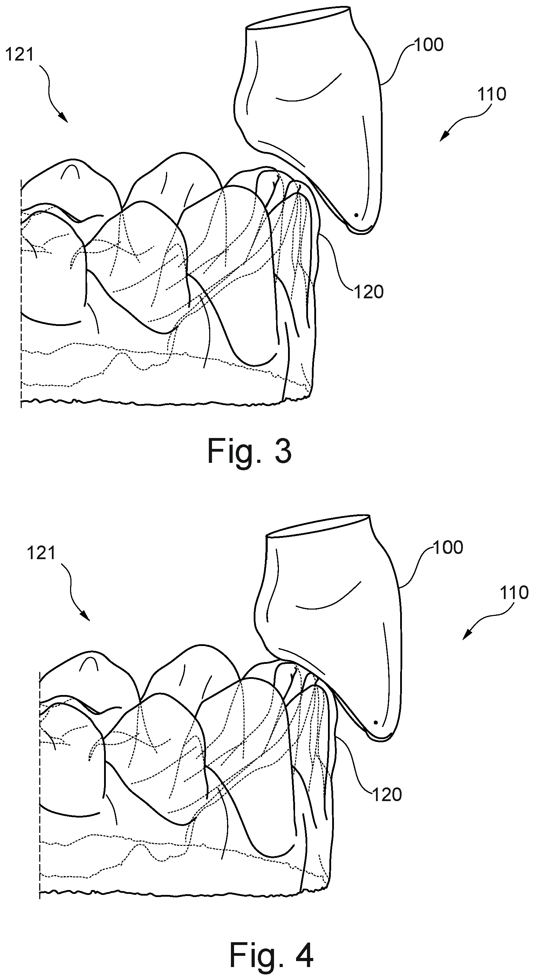

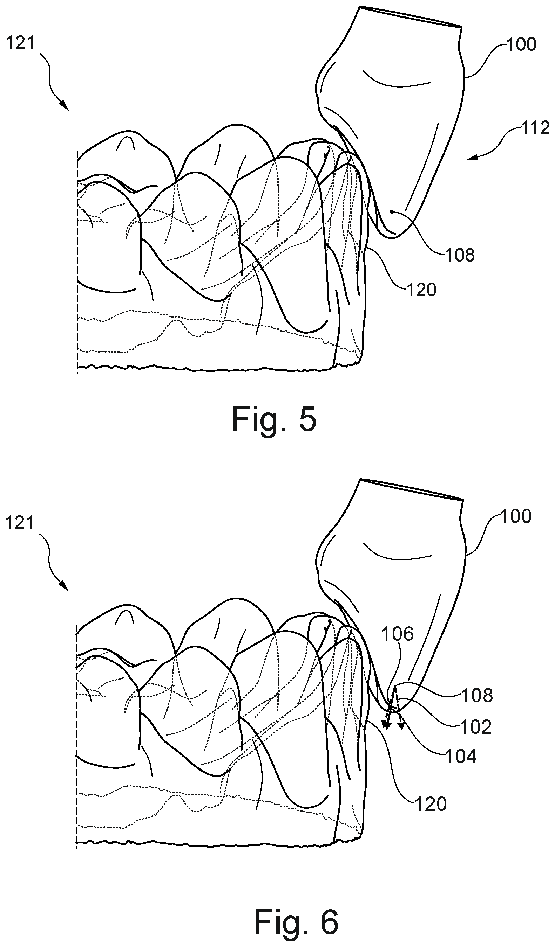

Fig. 3 shows an exemplary first three-dimensional digital dental model 100 of a first dental structure and an exemplary second three-dimensional digital dental model 120 of a second dental structure. In the example of Fig. 3, the first dental structure is a maxillary tooth, e.g., a maxillary incisor. The second dental structure is a mandibular antagonistic dental structure antagonistic to the first dental structure. For example, the antagonistic dental structure comprises a mandibular antagonist tooth to the first dental structure. The mandibular antagonist tooth may, e.g., be comprised by a mandibular dental arch 121. The first three-dimensional digital dental model 100 is arranged with an initial start orientation 110 relative to the second three-dimensional digital dental model 120. For example, when starting a positioning of the first three-dimensional digital dental model 100 relative to the second three-dimensional digital dental model 120, a definition of the start orientation 110 may be stored for further use. Fig. 4 and Fig. 5 illustrate the positioning of the first three-dimensional digital dental model 100 relative to the second three-dimensional digital dental model 120. The positioning may, e.g., comprise a moving of the first three-dimensional digital dental model 100 towards the second three-dimensional digital dental model 120. As illustrated in Fig. 4, the first three-dimensional digital dental model 100 may, e.g., be brought in contact with the second three-dimensional digital dental model 120.

The positioning of the first three-dimensional digital dental model 100 relative to the second three-dimensional digital dental model 120 may, e.g., be restricted by a boundary condition defining one or more distance criteria to be preserved between the first and second three-dimensional digital dental model 100, 120. The distance criteria may, e.g., define a threshold for a minimum distance between the first and second three-dimensional digital dental model 100, 120. For example, the threshold for the minimum distance may be a threshold of size zero. Thus, the first three-dimensional digital dental model 100 may, e.g., be brought in contact with the second three-dimensional digital dental model 120 as shown in Fig. 4, but the first three-dimensional digital dental model 100 may be prevented from being moved further into the second three-dimensional digital dental model 120. For example, the first three-dimensional digital dental model 100 may be prevented from being moved further into the second three-dimensional digital dental model 120 using a collision interaction, defining the two three-dimensional digital dental models 100, 120 as two rigid bodies.

A preserving of the distance criteria by the positioning may result in a rotating of the first three-dimensional digital dental model 100 as illustrated in Fig. 5. The first three-dimensional digital dental model 100 may be rotated around an axis 108 of rotation starting from the start orientation 110. By the rotating, an angle of a current orientation of the first three-dimensional digital dental model 100 relative to the initial start orientation of the first three-dimensional digital dental model 100 at a start of the rotation may be increased until the current orientation reaches a provisional end orientation 112 of the first three-dimensional digital dental model 100 at an end of the rotation as shown in Fig. 5. The provisional end orientation 112 may be rotated relative to the initial start orientation 110 by a maximum angle.

The positioning of the first three-dimensional digital dental model 100 may be adjusted as illustrated in Fig. 6 to Fig. 8. A preserving of the distance criteria by the adjusting of the transforming may result in a further rotating of the first three-dimensional digital dental model 100 around the axis 108 of rotation. The further rotating of the adjusting may at least partially reverse the rotating of the first three-dimensional digital dental model 100 such that the current angle relative to the start orientation 110 is decreased. For example, during the adjusting, the current angle may, e.g., be varied between the provisional end orientation 112 and the start orientation 110. The varying of the current angle may, e.g., comprise a temporary increasing.

As shown in Fig. 6, during the adjusting of the transforming, a first visual indicator 102, a second visual indicator 104, and a third visual indicator 106 may be displayed. The first visual indicator 102 may indicate the initial start orientation 110 of the first three-dimensional digital dental model 100 as a first reference orientation using the stored definition of the start orientation 110. For example, the first visual indicator 102 is a first arrow pointing in the first reference direction. The second visual indicator 104 may indicate the current orientation of the first three-dimensional digital dental model 100. For example, the second visual indicator 104 is a second arrow pointing in a direction of the current orientation of the first three-dimensional digital dental model 100 as a current direction. The third visual indicator 106 may indicate the provisional end orientation 112 as a second reference orientation using the stored definition of the provisional end orientation 112. For example, the third visual indicator 106 is a third arrow pointing in the second reference direction.

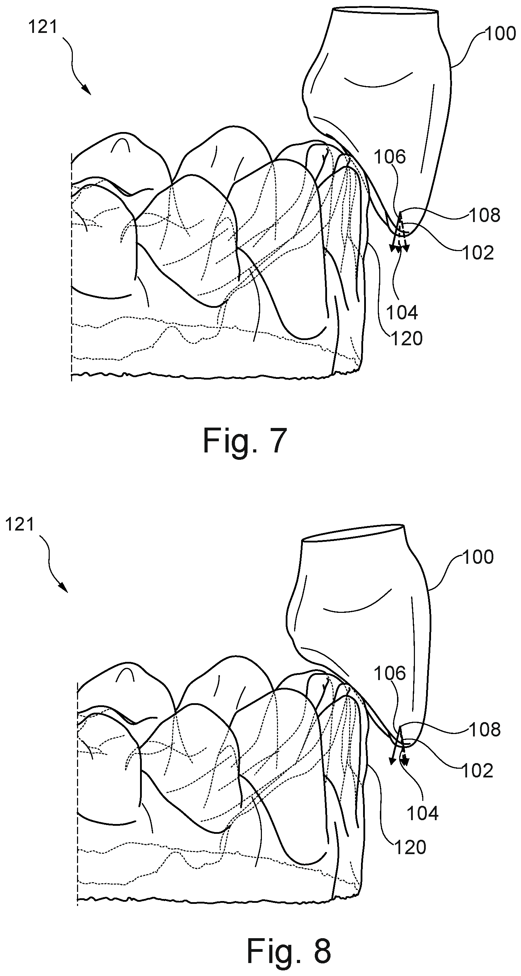

The second visual indicator 104 may, e.g., be used for assessing a relation between the current orientation of the first three-dimensional digital dental model 100 and the start as well as the provisional end orientation 110, 112 of the first three-dimensional digital dental model 100. Starting from the provisional end orientation 112, the first three-dimensional digital dental model 100 may be rotated back, i.e., the rotation of the positioning may be reversed, as illustrated in Fig. 6. Thereby, an angle between the third and second visual indicator 106, 104 may increase. During the back rotation, an intermediate state as illustrated in Fig. 7 may be reached with the current orientation of the first three-dimensional digital dental model 100 being an orientation between the start and provisional end orientation 110, 112. In this intermediate state, the back rotation may, e.g., be continued by rotating the current orientation towards the initial start orientation 110 or the angle relative to the start orientation 110 may be increased again by rotating the current orientation towards the provisional end orientation 112. As illustrated in Fig. 8, the back rotation may, e.g., be continued. Thereby, an angle between the second and first visual indicator 104, 102 may decrease. For example, the complete rotation of the positioning may be precisely undone.

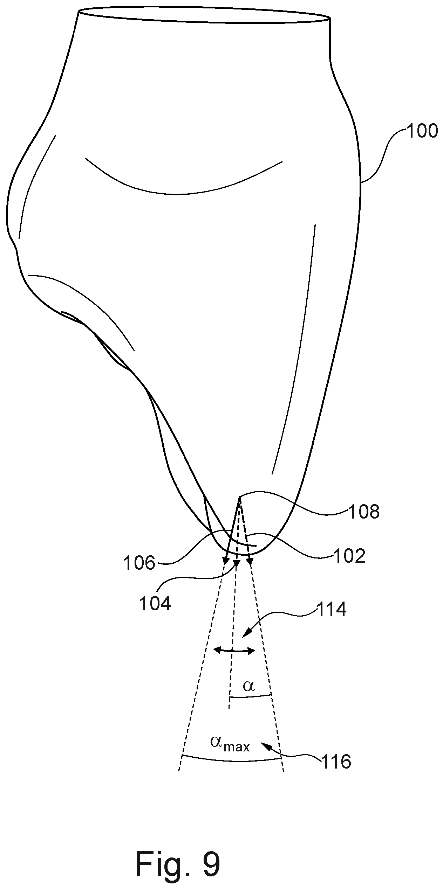

Fig. 9 shows visual indicators 102, 104, 106 of orientations of the first three-dimensional digital dental model 100 which may be displayed during an adjusting of a transforming, in particular positioning, of the first three-dimensional digital dental model 100. The second visual indicator 104, e.g., provided in form of an arrow pointing in a current direction of the current orientation of the first three-dimensional digital dental model 100, may indicate the current orientation of the first three-dimensional digital dental model 100. The current orientation of the first three-dimensional digital dental model 100 may be varied between the provisional end orientation indicated by the third visual indicator 106 and the initial start orientation indicated by the first visual indicator 102. By varying the current orientation of the first three-dimensional digital dental model 100, an angle α of the current orientation relative to the start orientation may be varied between the maximum value αₘₐₓ of the angle α reached by the provisional end orientation and 0, which corresponds to an alignment with the initial start orientation.

The initial start orientation 110 of the first three-dimensional digital dental model 100 may be indicated by the first visual indicator 102 as a first reference orientation using the stored definition of the start orientation, while the provisional end orientation 112 of the first three-dimensional digital dental model 100 may be indicated by the third visual indicator 106 as a second reference orientation using the stored definition of the provisional end orientation. These two reference directions indicated by the first and third visual indicator 102, 106 may be used for assessing a relation of the current orientation of the three-dimensional digital dental model 100 as indicated by the second visual indicator 104 to the start as well as provisional end orientation of the first three-dimensional digital dental model 100. The current orientation may be adjusted relative to the start and provisional end orientation of the first three-dimensional digital dental model 100. In particular, even the complete rotation of the positioning may, e.g., be precisely undone using the second visual indicator 104 to determine an alignment of the current orientation of the first three-dimensional digital dental model 100 with the initial start orientation.

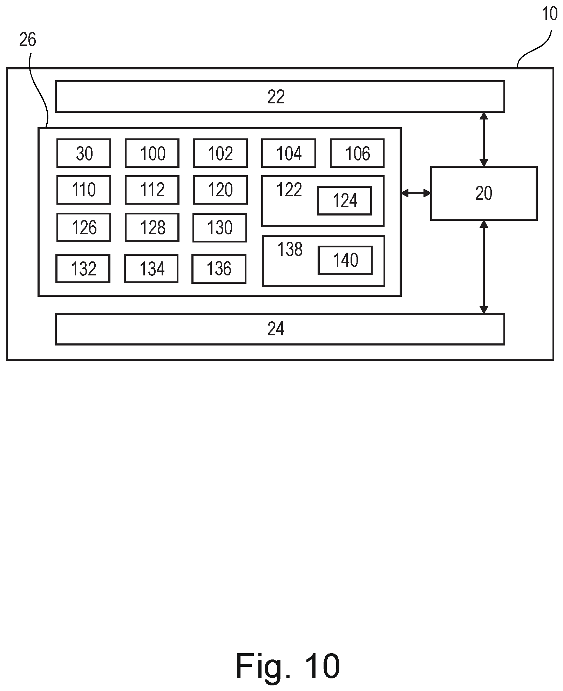

Fig. 10 shows an exemplary computer device 10 for adjusting a transformation of a first three-dimensional digital dental model 100 of a first dental structure relative to a second three-dimensional digital dental model 120 of a second dental structure. The exemplary computer device 10 may further be configured for generating a three-dimensional digital prosthesis model 140 of a dental prosthesis comprising the adjusted first three-dimensional digital dental model 100 and providing control data 138 for controlling a manufacturing of the dental prosthesis using a manufacturing device. The control data may, e.g., define the three-dimensional digital prosthesis model 140. The exemplary computer device 10 may further be configured for controlling the manufacturing device to manufacture the dental prosthesis as a physical copy of the three-dimensional digital prosthesis model 140 using the control data 138.

The exemplary computer device 10 is shown as comprising a processing unit 20. The processing unit 20 is intended to represent one or more processors or processing cores or other processing components configured for executing computational tasks. The processing unit 20 is shown as being connected to a hardware interface 22 and a memory unit 26. The hardware interface 22 may enable the processing unit 20 to exchange commands and data with other components, like a manufacturing device.

The computer device 10 is further shown as being connected to an optional user interface 24 which may, e.g., enable an operator to control and operate the computer device 10. The user interface 24 may, e.g., comprise an output and/or input device enabling the operator to interact with the computer device 10.

The memory unit 26 is shown as containing machine-executable program instructions 30. The machine-executable program instructions 30 enable the processing unit 20 to perform controlling tasks, like controlling a manufacturing device, to perform numerical tasks, as well as to perform various signal data processing tasks. The machine-executable program instructions 30 may, e.g., enable the processing unit 20 and thus the computer device 10 to adjust a transformation of a first three-dimensional digital dental model 100 of a first dental structure relative to a second three-dimensional digital dental model 120 of a second dental structure. The machine-executable program instructions 30 may, e.g., enable the processing unit 20 and thus the computer device 10 to generate the three-dimensional digital prosthesis model 140 and/or to control a manufacturing of a dental prosthesis using the control data 138 with the three-dimensional digital prosthesis model 140. The machine-executable program instructions 30 may, e.g., enable the processing unit 20 and thus the computer device 10 to execute any of the methods of Fig. 1 and 2.

For example, memory unit 26 is shown to comprise the first three-dimensional digital dental model 100 of the first dental structure and the second three-dimensional digital dental model 120 of the second dental structure. The computer device 10 may, e.g., be used for transforming the first three-dimensional digital dental model 100. The transforming may comprise a positioning of the first three-dimensional digital dental model 100 relative to the second three-dimensional digital dental model 120. For example, memory unit 26 is shown to comprise a definition of an initial start orientation 110 of the first three-dimensional digital dental model 100 at a start of the positioning and in particular at a start of a rotation of the first three-dimensional digital dental model 100 during the positioning. For example, memory unit 26 is shown to further comprise a definition of a provisional end orientation 112 of the first three-dimensional digital dental model 100 reached by the positioning. The memory unit 26 is shown to further, e.g., comprise a definition of a boundary condition 122 restricting the positioning of the first three-dimensional digital dental model 100. The boundary condition 122 may, e.g., define one or more distance criteria 124 to be preserved between the first and second three-dimensional digital dental model 100, 120.

The positioning of the first three-dimensional digital dental model 100 relative to the second three-dimensional digital dental model 120 may, e.g., be restricted by the boundary condition 122 defining the one or more distance criteria 124 to be preserved between the first and second three-dimensional digital dental model 100, 120. A preserving of the one or more distance criteria 124 by the positioning may, e.g., result in a rotating of the first three-dimensional digital dental model 100 around an axis 108 of rotation increasing an angle 114 of a current orientation of the first three-dimensional digital dental model 100 relative to an initial start orientation 110 of the first three-dimensional digital dental model 100 at a start of the rotation until the current orientation reaches a provisional end orientation 112 of the first three-dimensional digital dental model 100 at an end of the rotation. At this end of the rotation, the current orientation may reach a maximum angle 116 relative to the start orientation 110.

The transforming of the first three-dimensional digital dental model 100, in particular the positioning of the first three-dimensional digital dental model 100, may, e.g., be adjusted. A preserving of the one or more distance criteria 124 of the boundary condition 122 by the adjusting of the transforming may, e.g., result in a further rotating of the first three-dimensional digital dental model 100 around the axis 108 of rotation using the stored definition of the start orientation 110 as a first reference orientation. Furthermore, the provisional end orientation 112 may be stored as well and used as a second reference orientation for the further rotating. The further rotating may, e.g., at least partially reverse the rotating of the first three-dimensional digital dental model 100 such that the current angle 114 of the first three-dimensional digital dental model 100 relative to the start orientation 110 is decreased.

During the adjusting of the transforming, a first visual indicator 102, a second visual indicator 104, and a third visual indicator 106 may be displayed. The first visual indicator 102 may, e.g., indicate the initial start orientation 110 of the first three-dimensional digital dental model 100. For example, the first visual indicator 102 may be a first arrow pointing in a first reference direction. The second visual indicator 104 may, e.g., indicate the current orientation of the first three-dimensional digital dental model 100. For example, the second visual indicator 104 may be a second arrow pointing in a direction of the current orientation of the first three-dimensional digital dental model 100 as a current direction. The third visual indicator 106 may, e.g., indicate the provisional end orientation 112 of the first three-dimensional digital dental model 100. For example, the third visual indicator 106 is a third arrow pointing in the second reference direction.

The second visual indicator 104 may, e.g., be used for assessing a relation between the current orientation of the first three-dimensional digital dental model 100 and the start as well as the provisional end orientation 110, 112 of the first three-dimensional digital dental model 100. Starting from the provisional end orientation 112, the first three-dimensional digital dental model 100 may be rotated back, i.e., the rotation of the positioning may be reversed. Thereby, an angle between the third and second visual indicator 106, 104 may increase. Due to the back rotation, an angle 114 between the second and first visual indicator 104, 102 may decrease. For example, the complete rotation of the positioning may be precisely undone.

For example, the definition of the start orientation is stored upon satisfying a first storing criterion 126. For example, the first storing criterion 126 is a starting of the positioning. For example, the definition of the start orientation is stored automatically upon starting of the positioning. For example, the first storing criterion 126 is a starting of the rotation. For example, the definition of the start orientation is stored automatically upon starting of the rotation. For example, the first storing criterion 126 is a detecting of a pressing of an activation button.

For example, the method further comprises a deleting of the definition of the start orientation upon satisfying a first deleting criterion 128. For example, the first deleting criterion 128 is an ending of the adjusting of the transforming. For example, the definition of the start orientation is deleted automatically upon ending of the adjusting of the transforming. For example, the first deleting criterion 128 is a detecting of a pressing of the activation button. For example, the first deleting criterion 128 is a detecting of a releasing of the activation button.

For example, the definition of the provisional end orientation is stored upon satisfying a second storing criterion 130. For example, the second storing criterion 130 is a starting of a decreasing of the current angle relative to the start orientation. For example, the method further comprises an updating of the definition of the provisional end orientation. For the updating, a turning orientation of the first three-dimensional digital dental model reached at a turning point during the adjusting of the transforming may be used. The turning point is a point at which the adjusting turns from a temporary increasing of the angle relative to the start orientation to a temporary decreasing. When a reaching of a turning point is detected, the stored provisional end orientation may be updated, i.e., replaced by the turning orientation of the first three-dimensional digital dental model reached at a turning point.

For example, the method further comprises a deleting of the definition of the start orientation upon satisfying a first deleting criterion. For example, the first deleting criterion is an ending of the adjusting of the transforming. For example, the definition of the start orientation is deleted automatically upon ending of the adjusting of the transforming. For example, the first deleting criterion is a detecting of a pressing of the activation button. For example, the first deleting criterion is a detecting of a releasing of the activation button.

For example, the method further comprises a deleting of the definition of the provisional end orientation upon satisfying a second deleting criterion 132. For example, the second deleting criterion 132 is an ending of the adjusting of the transforming. For example, the definition of the provisional end orientation is deleted automatically upon ending of the adjusting of the transforming. For example, the second deleting criterion 132 is a pressing of the activation button. For example, the second deleting criterion 132 is a releasing of the activation button.

For example, the visual indicators 102, 104, 106 are displayed upon satisfying a displaying criterion 134. For example, the displaying criterion 134 is a starting of a decreasing of the angle of rotation around the axis of rotation. For example, the displaying of the visual indicators 102, 104, 106 is terminated upon satisfying a termination criterion 136. For example, the termination criterion 136 is an ending of the adjusting of the transforming. For example, the termination criterion 136 is a detecting of a pressing of an activation button. For example, the termination criterion 136 is a detecting of a releasing of the activation button.

Finally, the three-dimensional digital prosthesis model 140 of the dental prosthesis comprising the adjusted first three-dimensional digital dental model 100 may be generated. Control data 138 for controlling a manufacturing of the dental prosthesis using a manufacturing device may be provided. The control data 138 may, e.g., define the three-dimensional digital prosthesis model 140. For example, the control data 138 may be used for controlling the manufacturing device to manufacture the dental prosthesis. For example, the dental prosthesis is manufactured as a physical copy of the three-dimensional digital prosthesis model 140 provided by the control data 138.

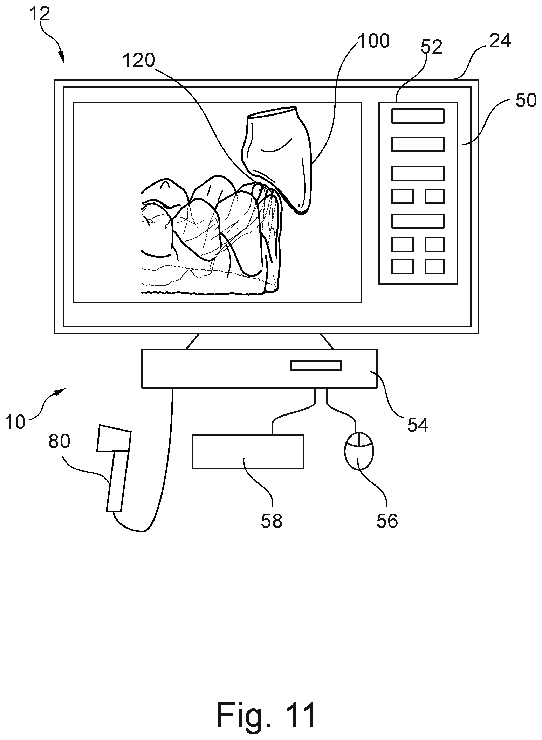

Fig. 11 shows an exemplary computer system 12 with a computer device 10. The computer device 10 is configured for adjusting a transformation of a first three-dimensional digital dental model 100 of a first dental structure relative to a second three-dimensional digital dental model 120 of a second dental structure. The computer device 10 may, e.g., be configured as shown in Fig. 10. The computer device 10 may comprise a hardware component 54 comprising one or more processing units as well as a memory storing machine-executable program instructions. Execution of the program instructions by the one or more processing units may cause the one or more processing units to control the computer device 10 to adjust the transforming of the first three-dimensional digital dental model 100 relative to the second three-dimensional digital dental model 120.

The computer system 12 may further comprise one or more input devices, like a keyboard 56 and a mouse 58, enabling a user to interact with the computer device 10. The one or more input devices may enable the user to control the adjusting of the transforming of the first three-dimensional digital dental model 100 relative to the second three-dimensional digital dental model 120. Furthermore, the computer system 12 may comprise one or more output devices of a user interface 24, like a display providing a graphical user interface 50 with control elements 52, e.g., GUI elements, enabling a visualization of the adjusting of the transforming of the first three-dimensional digital dental model 100 relative to the second three-dimensional digital dental model 120 for the user.

The computer system 12 may further comprise a scanner 80, e.g., an optical scanner, configured for acquiring scan data of dental structures of a patient. The scanner 80 may, e.g., be an intraoral scanner. The scan data acquired by the scanner 80 may, e.g., be used for providing the first and/or second three-dimensional digital dental model.

Furthermore, the computer device 10 of the computer system 12 may, e.g., be configured for generating a three-dimensional digital prosthesis model of a dental prosthesis comprising the adjusted first three-dimensional digital dental model 100 and providing control data for controlling a manufacturing of the dental prosthesis using a manufacturing device. The control data may, e.g., define the three-dimensional digital prosthesis model.

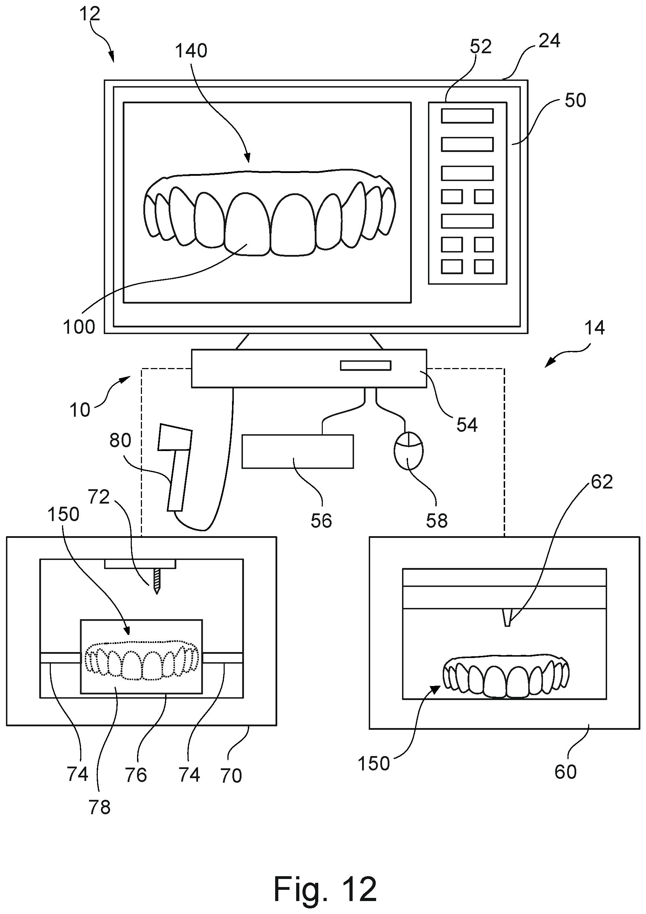

Fig. 12 shows an exemplary manufacturing system 14 for manufacturing a dental prosthesis 150. The manufacturing system 14 may, e.g., comprise the computer system 12 of Fig. 11 with the computer device 10 of Fig. 10 and/or Fig. 11. The computer device 10 of the computer system 12 may, e.g., be configured for controlling a manufacturing of the dental prosthesis 150 using one or more manufacturing devices 60, 70 comprised by the manufacturing system 14. For controlling the manufacturing of the dental prosthesis 150, the computer device 10 may use control data. The control data may, e.g., define a three-dimensional digital prosthesis model 140 of a dental prosthesis 150. The three-dimensional digital prosthesis model 140 may, e.g., comprise an adjusted first three-dimensional digital dental model. The dental prosthesis 150 may, e.g., be manufactured as a physical copy of the three-dimensional digital prosthesis model 140.

The manufacturing system 14 may comprise one or more manufacturing devices 60, 70. The one or more manufacturing devices 60, 70 may, e.g., be controlled by the computer device 10. For example, the manufacturing system 14 may comprise a manufacturing device in form of a machining device 70 controlled by the computer device 10. The machining device 70 may be configured to process a blank 76 using one or more machining tools 72. The blank 76 of raw material 78 may, e.g., be provided using a holding device 74 and cut into a desired shape and size of the element to be manufactured, e.g., the dental prosthesis 150 as defined by the three-dimensional digital prosthesis model 140. The machining device 70 is, e.g., a milling device with the machining tool 72 being a milling tool.

For example, the manufacturing system 14 may comprise a manufacturing device in form of a 3D printing device 60. The 3D printing device 60 may, e.g., be controlled by the computer device 10 and configured to print an element to be manufactured, e.g., the dental prosthesis 150 as defined by the three-dimensional digital prosthesis model 140. The 3D printing device 60 may, e.g., comprise a printing element 62 configured to print the respective element, e.g., the dental prosthesis 150, layer by layer. The printing element 62 may, e.g., comprise a nozzle configured for distributing printing material.

In case the element to be manufactured using the 3D printing device 60 is made using metal, the 3D printing device 60 may, e.g., be configured for executing selective laser sintering or melting. Selective laser sintering uses a laser for sintering a powdered material, aiming the laser automatically at points in space defined by a three-dimensional digital model of the element to be printed. The laser energy may result in a local sintering or melting of the powdered material, binding the material together to create a solid structure. For example, the printing element 62 of the 3D printing device 60 may comprise a laser and/or a distributing device for distributing the powdered material.

For example, the three-dimensional digital prosthesis model 140 may be used as a positive to define a negative of the dental prosthesis 150 in form of a negative three-dimensional digital prosthesis model. The negative three-dimensional digital prosthesis model may be used to manufacture, e.g., using machining device 70 or 3D printing device 60, one or more casting matrices. The one or more casting matrices may, e.g., be configured for casting the dental prosthesis 150 by inserting casting material into the casting matrix and curing the inserted casting material.

For example, a combination of manufacturing devices, e.g., machining device 70 or 3D printing device 60, may be used for manufacturing the dental prosthesis 150.

While the invention has been illustrated and described in detail in the drawings and foregoing description, such illustration and description are to be considered illustrative or exemplary and not restrictive; the invention is not limited to the disclosed examples.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

A single processor or other unit may fulfill the functions of several items recited in the claims. A computer program may be stored/distributed on a suitable medium, such as an optical storage medium or a solid-state medium supplied together with or as part of other hardware, but may also be distributed in other forms, such as via the Internet or other wired or wireless telecommunication systems.

As will be appreciated by one skilled in the art, aspects of the present invention may be embodied as an apparatus, method, computer program or computer program product. Accordingly, aspects of the present invention may take the form of an entirely hardware embodiment, an entirely software embodiment (including firmware, resident software, microcode, etc.) or an embodiment combining software and hardware aspects that may all generally be referred to herein as a "circuit," "module" or "system." Furthermore, aspects of the present invention may take the form of a computer program product embodied in one or more computer-readable medium(s) having computer executable code embodied thereon. A computer program comprises the computer executable code or "program instructions".

Any combination of one or more computer-readable medium(s) may be utilized. The computer-readable medium may be a computer-readable signal medium or a computer-readable storage medium. A "computer-readable storage medium" as used herein encompasses any tangible storage medium which may store instructions which are executable by a processor of a computer device. The computer-readable storage medium may be referred to as a computer-readable non-transitory storage medium. The computer-readable storage medium may also be referred to as a tangible computer-readable medium. For example, a computer-readable storage medium may also be able to store data which is able to be accessed by the processor of the computer device. Examples of computer-readable storage media include, but are not limited to: a floppy disk, a magnetic hard disk drive, a solid-state hard disk, flash memory, a USB thumb drive, Random Access Memory (RAM), Read Only Memory (ROM), an optical disk, a magneto-optical disk, and the register file of the processor. Examples of optical disks include Compact Disks (CD) and Digital Versatile Disks (DVD), for example CD-ROM, CD-RW, CD-R, DVD-ROM, DVD-RW, or DVD-R disks. A further example of an optical disk may be a Blu-ray disk. The term computer-readable storage medium also refers to various types of recording media capable of being accessed by the computer device via a network or communication link. For example, data may be retrieved over a modem, over the internet, or over a local area network. Computer executable code embodied on a computer-readable medium may be transmitted using any appropriate medium, including but not limited to wireless, wireline, optical fiber cable, RF, etc., or any suitable combination of the foregoing.

A computer-readable signal medium may include a propagated data signal with computer executable code embodied therein, for example, in baseband or as part of a carrier wave. Such a propagated signal may take any of a variety of forms, including, but not limited to, electromagnetic, optical, or any suitable combination thereof. A computer-readable signal medium may be any computer-readable medium that is not a computer-readable storage medium and that can communicate, propagate, or transport a program for use by or in connection with an instruction execution system, apparatus, or device.

"Computer memory" or "memory unit" is an example of a computer-readable storage medium. Computer memory is any memory which is directly accessible to a processing unit. "Computer storage" or "storage" is a further example of a computer-readable storage medium. Computer storage is any non-volatile computer-readable storage medium. For example, computer storage may also be computer memory or vice versa.

A "processor" or "processing unit" as used herein encompasses an electronic component which is able to execute a program or machine executable instruction or computer executable code. References to the computer device comprising "a processor" should be interpreted as possibly containing more than one processor or processing core. The processor may for instance be a multi-core processor. A processor may also refer to a collection of processors within a single computer device or distributed amongst multiple computer devices. The term computer device should also be interpreted to possibly refer to a collection or network of computer devices each comprising a processor or processors. The computer executable code may be executed by multiple processors that may be within the same computer device, or which may even be distributed across multiple computer devices.

Computer executable code may comprise machine executable instructions or a program which causes a processor to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Generally, the program instructions can be executed on one processor or on several processors. In the case of multiple processors, they can be distributed over several different entities like clients, servers, etc. Each processor could execute a portion of the instructions intended for that entity. Thus, when referring to a system or process involving multiple entities, the computer program or program instructions are understood to be adapted to be executed by a processor associated or related to the respective entity.

A "user interface" as used herein is an interface which allows a user or operator to interact with a computer or computer device. A 'user interface' may also be referred to as a 'human interface device." A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, gear sticks, steering wheel, pedals, wired glove, dance pad, remote control, one or more switches, one or more buttons, and an accelerometer are all examples of user interface components which enable the receiving of information or data from an operator.

A GUI element is a data object some of whose attributes specify the shape, layout and/or behavior of an area displayed on a graphical user interface, e.g., a screen. A GUI element can be a standard GUI element such as a button, a text box, a tab, an icon, a text field, a pane, a check-box item or item group or the like. A GUI element can likewise be an image, an alphanumeric character, or any combination thereof. At least some of the properties of the displayed GUI elements depend on the data value aggregated on the group of data objects said GUI element represents.

Aspects of the present invention are described with reference to flowchart illustrations and/or block diagrams of methods, apparatus (systems) and computer program products. It will be understood that each block or a portion of the blocks of the flowchart, illustrations, and/or block diagrams, can be implemented by computer program instructions in the form of computer executable code when applicable. It is further understood that, when not mutually exclusive, combinations of blocks in different flowcharts, illustrations, and/or block diagrams may be combined. These computer program instructions may be provided to a processor of a general-purpose computer, special-purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer-readable medium that can direct a computer, other programmable data processing apparatus, or other devices to function in a particular manner, such that the instructions stored in the computer-readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer-implemented process such that the instructions which execute on the computer or other programmable apparatus provide processes for implementing the functions/acts specified in the flowchart and/or block diagram block or blocks.

Although the invention may have been described with reference to specific examples, it should be understood that the invention is not limited to these examples only and that many variations of these examples may be readily envisioned by the skilled person after having read the present disclosure. The invention may thus further be described without limitation and by way of example only by the following embodiments. The following exemplary embodiments may contain preferred embodiments. Accordingly, the term "feature combination" as used therein may refer to such a "preferred embodiment".
1. A computer-implemented method for adjusting a transformation of a first three-dimensional digital dental model of a first dental structure relative to a second three-dimensional digital dental model of a second dental structure,
   the method comprising:
   receiving the first three-dimensional digital dental model;
   receiving the second three-dimensional digital dental model;
   transforming the first three-dimensional digital dental model, the transforming comprising a positioning of the first three-dimensional digital dental model relative to the second three-dimensional digital dental model, the positioning of the first three-dimensional digital dental model relative to the second three-dimensional digital dental model being restricted by a boundary condition defining one or more distance criteria to be preserved between the first and second three-dimensional digital dental model, a preserving of the one or more distance criteria by the positioning resulting in a rotating of the first three-dimensional digital dental model around an axis of rotation increasing an angle of a current orientation of the first three-dimensional digital dental model relative to an initial start orientation of the first three-dimensional digital dental model at a start of the rotation until the current orientation reaches a provisional end orientation of the first three-dimensional digital dental model at an end of the rotation with a maximum angle relative to the start orientation, a definition of the start orientation being stored for further use; and
   adjusting the transforming of the first three-dimensional digital dental model, a preserving of the one or more distance criteria by the adjusting of the transforming resulting in a further rotating of the first three-dimensional digital dental model around the axis of rotation using the stored definition of the start orientation as a first reference orientation, the further rotating at least partially reversing the rotating of the first three-dimensional digital dental model such that the current angle relative to the start orientation is decreased.
2. The method of feature combination 1, the one or more distance criteria defining a threshold for a minimum distance between the first and second three-dimensional digital dental model.
3. The method of feature combination 2, the threshold for the minimum distance being one of the following: a threshold of size zero; a threshold of positive size; a threshold of negative size.
4. The method of any of the previous feature combinations, the one or more distance criteria defining a fixed minimum distance to be enforced between the first and second three-dimensional digital dental model.
5. The method of feature combination 4, the fixed minimum distance being one of the following: a fixed minimum distance of size zero; a fixed minimum distance of positive size; a fixed minimum distance of negative size.
6. The method of any of the previous feature combinations, the method further comprising displaying, during the adjusting of the transforming, a first visual indicator indicating the start orientation as a first reference orientation using the stored definition of the start orientation.
7. The method of feature combination 6, the first visual indicator indicating a direction of the start orientation of the first three-dimensional digital dental model as a first reference direction.
8. The method of feature combination 7, the first visual indicator comprising a first segment pointing in the first reference direction.
9. The method of any of the previous feature combinations, the method further comprising displaying, during the adjusting of the transforming, a second visual indicator indicating the current orientation of the first three-dimensional digital dental model.
10. The method of feature combination 9, the second visual indicator indicating a direction of the current orientation of the first three-dimensional digital dental model during the adjusting as a current direction.
11. The method of feature combination 10, the second visual indicator comprising a second segment pointing in the current direction.
12. The method of any of the previous feature combinations, the method further comprising storing a definition of the provisional end orientation for further use, during the adjusting of the transforming, a third visual indicator indicating the provisional end orientation as a second reference orientation being displayed using the stored definition of the provisional end orientation.
13. The method of feature combination 12, the third visual indicator indicating a direction of the provisional end orientation of the first three-dimensional digital dental model as a second reference direction.
14. The method of feature combination 13, the third visual indicator comprising a third segment pointing in the second reference direction.
15. The method of any of the feature combinations 12 to 14, the method further comprising an updating of the definition of the provisional end orientation using a turning orientation of the first three-dimensional digital dental model reached at a turning point during the adjusting of the transforming, the turning point being a point at which the adjusting turns from a temporary increasing of the angle relative to the start orientation to a temporary decreasing.
16. The method of any of the feature combinations 12 to 15, the adjusting of the transforming of the first three-dimensional digital dental model comprising an interpolation of the current orientation of the first three-dimensional digital dental model using the definition of the start orientation and the definition of the provisional end orientation.
17. The method of any of the previous feature combinations, the method further comprising:
   generating a three-dimensional digital prosthesis model of a dental prosthesis comprising the adjusted first three-dimensional digital dental model; and
   providing control data for controlling a manufacturing of the dental prosthesis using a manufacturing device, the control data defining the three-dimensional digital prosthesis model.
18. A computer program for adjusting a transformation of a first three-dimensional digital dental model of a first dental structure relative to a second three-dimensional digital dental model of a second dental structure,
   the computer program comprising program instructions being executable by a processing unit of a computer device to cause the computer device to:
   receive the first three-dimensional digital dental model;
   receive the second three-dimensional digital dental model;
   transform the first three-dimensional digital dental model, the transforming comprising a positioning of the first three-dimensional digital dental model relative to the second three-dimensional digital dental model, the positioning of the first three-dimensional digital dental model relative to the second three-dimensional digital dental model being restricted by a boundary condition defining one or more distance criteria to be preserved between the first and second three-dimensional digital dental model, a preserving of the one or more distance criteria by the positioning resulting in a rotating of the first three-dimensional digital dental model around an axis of rotation increasing an angle of a current orientation of the first three-dimensional digital dental model relative to an initial start orientation of the first three-dimensional digital dental model at a start of the rotation until the current orientation reaches a provisional end orientation of the first three-dimensional digital dental model at an end of the rotation with a maximum angle relative to the start orientation, a definition of the start orientation being stored for further use; and
   adjust the transforming of the first three-dimensional digital dental model, a preserving of the one or more distance criteria by the adjusting of the transforming resulting in a further rotating of the first three-dimensional digital dental model around the axis of rotation using the stored definition of the start orientation as a first reference orientation, the further rotating at least partially reversing the rotating of the first three-dimensional digital dental model such that the current angle relative to the start orientation is decreased.
19. A computer program product for adjusting a transformation of a first three-dimensional digital dental model of a first dental structure relative to a second three-dimensional digital dental model of a second dental structure,
   the computer program product comprising a non-transitory computer-readable storage medium having program instructions embodied therewith, the program instructions being executable by a processing unit of a computer device to cause the computer device to:
   receive the first three-dimensional digital dental model;
   receive the second three-dimensional digital dental model;
   transform the first three-dimensional digital dental model, the transforming comprising a positioning of the first three-dimensional digital dental model relative to the second three-dimensional digital dental model, the positioning of the first three-dimensional digital dental model relative to the second three-dimensional digital dental model being restricted by a boundary condition defining one or more distance criteria to be preserved between the first and second three-dimensional digital dental model, a preserving of the one or more distance criteria by the positioning resulting in a rotating of the first three-dimensional digital dental model around an axis of rotation increasing an angle of a current orientation of the first three-dimensional digital dental model relative to an initial start orientation of the first three-dimensional digital dental model at a start of the rotation until the current orientation reaches a provisional end orientation of the first three-dimensional digital dental model at an end of the rotation with a maximum angle relative to the start orientation, a definition of the start orientation being stored for further use; and
   adjust the transforming of the first three-dimensional digital dental model, a preserving of the one or more distance criteria by the adjusting of the transforming resulting in a further rotating of the first three-dimensional digital dental model around the axis of rotation using the stored definition of the start orientation as a first reference orientation, the further rotating at least partially reversing the rotating of the first three-dimensional digital dental model such that the current angle relative to the start orientation is decreased.
20. A computer device for adjusting a transformation of a first three-dimensional digital dental model of a first dental structure relative to a second three-dimensional digital dental model of a second dental structure,
   the computer device comprising a processing unit and a memory unit storing program instructions executable by the processing unit, an execution of the program instructions by the processing unit causing the computer device to:
   receive the first three-dimensional digital dental model;
   receive the second three-dimensional digital dental model;
   transform the first three-dimensional digital dental model, the transforming comprising a positioning of the first three-dimensional digital dental model relative to the second three-dimensional digital dental model, the positioning of the first three-dimensional digital dental model relative to the second three-dimensional digital dental model being restricted by a boundary condition defining one or more distance criteria to be preserved between the first and second three-dimensional digital dental model, a preserving of the one or more distance criteria by the positioning resulting in a rotating of the first three-dimensional digital dental model around an axis of rotation increasing an angle of a current orientation of the first three-dimensional digital dental model relative to an initial start orientation of the first three-dimensional digital dental model at a start of the rotation until the current orientation reaches a provisional end orientation of the first three-dimensional digital dental model at an end of the rotation with a maximum angle relative to the start orientation, a definition of the start orientation being stored for further use; and
   adjust the transforming of the first three-dimensional digital dental model, a preserving of the one or more distance criteria by the adjusting of the transforming resulting in a further rotating of the first three-dimensional digital dental model around the axis of rotation using the stored definition of the start orientation as a first reference orientation, the further rotating at least partially reversing the rotating of the first three-dimensional digital dental model such that the current angle relative to the start orientation is decreased.
21. A manufacturing system comprising the computer device of feature combination 20, the manufacturing system further comprising a manufacturing device configured for manufacturing a dental prosthesis,
   execution of the program instructions by the processing unit further causing the computer device to:
   generate a three-dimensional digital prosthesis model of the dental prosthesis comprising the adjusted first three-dimensional digital dental model;
   provide control data for controlling the manufacturing of the dental prosthesis using the manufacturing device, the control data defining the three-dimensional digital prosthesis model; and
   control the manufacturing device to manufacture the dental prosthesis using the control data, the dental prosthesis being manufactured as a physical copy of the three-dimensional digital prosthesis model.

### REFERENCE SIGNS LIST

- 10: computer device
- 12: computer system
- 14: manufacturing system
- 20: processing unit
- 22: hardware interface
- 24: user interface
- 26: memory unit
- 30: program instructions
- 50: graphical user interface
- 52: control elements
- 54: hardware component
- 56: keyboard
- 58: mouse
- 60: 3D printing device
- 62: printing element
- 70: machining device
- 72: machining tool
- 74: holding device
- 76: blank
- 78: raw material
- 80: scanner
- 100: first 3D digital dental model
- 102: first visual indicator
- 104: second visual indicator
- 106: third visual indicator
- 108: axis
- 110: start orientation
- 112: provisional end orientation
- 114: angle
- 116: maximum angle
- 120: second 3D digital dental model
- 121: dental arch
- 122: boundary condition
- 124: distance criteria
- 126: first storing criterion
- 128: first deleting criterion
- 130: second storing criterion
- 132: second deleting criterion
- 134: displaying criterion
- 136: termination criterion
- 138: control data
- 140: 3D digital prosthesis model
- 150: dental prosthesis

## Claims

1. A computer-implemented method for adjusting a transformation of a first three-dimensional digital dental model (100) of a first dental structure relative to a second three-dimensional digital dental model (120) of a second dental structure,
the method comprising:
receiving the first three-dimensional digital dental model (100);
receiving the second three-dimensional digital dental model (120);
transforming the first three-dimensional digital dental model (100), the transforming comprising a positioning of the first three-dimensional digital dental model (100) relative to the second three-dimensional digital dental model (120), the positioning of the first three-dimensional digital dental model (100) relative to the second three-dimensional digital dental model (120) being restricted by a boundary condition (122) defining one or more distance criteria (124) to be preserved between the first and second three-dimensional digital dental model (100; 120), a preserving of the one or more distance criteria (124) by the positioning resulting in a rotating of the first three-dimensional digital dental model (100) around an axis (108) of rotation increasing an angle (114) of a current orientation of the first three-dimensional digital dental model (100) relative to an initial start orientation (110) of the first three-dimensional digital dental model (100) at a start of the rotation until the current orientation reaches a provisional end orientation (112) of the first three-dimensional digital dental model (100) at an end of the rotation with a maximum angle (116) relative to the start orientation (110), a definition of the start orientation (110) being stored for further use; and
adjusting the transforming of the first three-dimensional digital dental model (100), a preserving of the one or more distance criteria (124) by the adjusting of the transforming resulting in a further rotating of the first three-dimensional digital dental model (100) around the axis (108) of rotation using the stored definition of the start orientation (110) as a first reference orientation, the further rotating at least partially reversing the rotating of the first three-dimensional digital dental model (100) such that the current angle (114) relative to the start orientation (110) is decreased.

2. The method of claim 1, the one or more distance criteria (124) defining a threshold for a minimum distance between the first and second three-dimensional digital dental model (100; 120),
the threshold for the minimum distance preferably being one of the following: a threshold of size zero; a threshold of positive size; a threshold of negative size.

3. The method of any of the previous claims, the one or more distance criteria (124) defining a fixed minimum distance to be enforced between the first and second three-dimensional digital dental model (100; 120),
the fixed minimum distance preferably being one of the following: a fixed minimum distance of size zero; a fixed minimum distance of positive size; a fixed minimum distance of negative size.

4. The method of any of the previous claims, the method further comprising displaying, during the adjusting of the transforming, a first visual indicator (102) indicating the start orientation (110) as a first reference orientation using the stored definition of the start orientation (110).

5. The method of claim 4, the first visual indicator (102) indicating a direction of the start orientation (110) of the first three-dimensional digital dental model (100) as a first reference direction,
the first visual indicator (102) preferably comprising a first segment pointing in the first reference direction.

6. The method of any of the previous claims, the method further comprising displaying, during the adjusting of the transforming, a second visual indicator (104) indicating the current orientation of the first three-dimensional digital dental model (100).

7. The method of claim 6, the second visual indicator (104) indicating a direction of the current orientation of the first three-dimensional digital dental model (100) during the adjusting as a current direction,
the second visual indicator (104) preferably comprising a second segment pointing in the current direction.

8. The method of any of the previous claims, the method further comprising storing a definition of the provisional end orientation (112) for further use, during the adjusting of the transforming, a third visual indicator (106) indicating the provisional end orientation (112) as a second reference orientation being displayed using the stored definition of the provisional end orientation (112).

9. The method of claim 8, the third visual indicator (106) indicating a direction of the provisional end orientation (112) of the first three-dimensional digital dental model (100) as a second reference direction,
the third visual indicator (106) preferably comprising a third segment pointing in the second reference direction.

10. The method of any of claims 8 to 9, the method further comprising an updating of the definition of the provisional end orientation (112) using a turning orientation of the first three-dimensional digital dental model (100) reached at a turning point during the adjusting of the transforming, the turning point being a point at which the adjusting turns from a temporary increasing of the angle (114) relative to the start orientation (110) to a temporary decreasing, and/or
the adjusting of the transforming of the first three-dimensional digital dental model (100) comprising an interpolation of the current orientation of the first three-dimensional digital dental model (100) using the definition of the start orientation (110) and the definition of the provisional end orientation (112).

11. The method of any of the previous claims, the method further comprising:
generating a three-dimensional digital prosthesis model (140) of a dental prosthesis (150) comprising the adjusted first three-dimensional digital dental model (100); and
providing control data (138) for controlling a manufacturing of the dental prosthesis (150) using a manufacturing device (60, 70), the control data (138) defining the three-dimensional digital prosthesis model (140).

12. A computer program for adjusting a transformation of a first three-dimensional digital dental model (100) of a first dental structure relative to a second three-dimensional digital dental model (120) of a second dental structure,
the computer program comprising program instructions (30) being executable by a processing unit (20) of a computer device (10) to cause the computer device (10) to:
receive the first three-dimensional digital dental model (100);
receive the second three-dimensional digital dental model (120);
transform the first three-dimensional digital dental model (100), the transforming comprising a positioning of the first three-dimensional digital dental model (100) relative to the second three-dimensional digital dental model (120), the positioning of the first three-dimensional digital dental model (100) relative to the second three-dimensional digital dental model (120) being restricted by a boundary condition (122) defining one or more distance criteria (124) to be preserved between the first and second three-dimensional digital dental model (100; 120), a preserving of the one or more distance criteria (124) by the positioning resulting in a rotating of the first three-dimensional digital dental model (100) around an axis (108) of rotation increasing an angle (114) of a current orientation of the first three-dimensional digital dental model (100) relative to an initial start orientation (110) of the first three-dimensional digital dental model (100) at a start of the rotation until the current orientation reaches a provisional end orientation (112) of the first three-dimensional digital dental model (100) at an end of the rotation with a maximum angle (116) relative to the start orientation (110), a definition of the start orientation (110) being stored for further use; and
adjust the transforming of the first three-dimensional digital dental model (100), a preserving of the one or more distance criteria (124) by the adjusting of the transforming resulting in a further rotating of the first three-dimensional digital dental model (100) around the axis (108) of rotation using the stored definition of the start orientation (110) as a first reference orientation, the further rotating at least partially reversing the rotating of the first three-dimensional digital dental model (100) such that the current angle (114) relative to the start orientation (110) is decreased.

13. A computer program product for adjusting a transformation of a first three-dimensional digital dental model (100) of a first dental structure relative to a second three-dimensional digital dental model (120) of a second dental structure,
the computer program product comprising a non-transitory computer-readable storage medium having program instructions (30) embodied therewith, the program instructions (30) being executable by a processing unit (20) of a computer device (10) to cause the computer device (10) to:
receive the first three-dimensional digital dental model (100);
receive the second three-dimensional digital dental model (120);
transform the first three-dimensional digital dental model (100), the transforming comprising a positioning of the first three-dimensional digital dental model (100) relative to the second three-dimensional digital dental model (120), the positioning of the first three-dimensional digital dental model (100) relative to the second three-dimensional digital dental model (120) being restricted by a boundary condition (122) defining one or more distance criteria (124) to be preserved between the first and second three-dimensional digital dental model (100; 120), a preserving of the one or more distance criteria (124) by the positioning resulting in a rotating of the first three-dimensional digital dental model (100) around an axis (108) of rotation increasing an angle (114) of a current orientation of the first three-dimensional digital dental model (100) relative to an initial start orientation (110) of the first three-dimensional digital dental model (100) at a start of the rotation until the current orientation reaches a provisional end orientation (112) of the first three-dimensional digital dental model (100) at an end of the rotation with a maximum angle (116) relative to the start orientation (110), a definition of the start orientation (110) being stored for further use; and
adjust the transforming of the first three-dimensional digital dental model (100), a preserving of the one or more distance criteria (124) by the adjusting of the transforming resulting in a further rotating of the first three-dimensional digital dental model (100) around the axis (108) of rotation using the stored definition of the start orientation (110) as a first reference orientation, the further rotating at least partially reversing the rotating of the first three-dimensional digital dental model (100) such that the current angle (114) relative to the start orientation (110) is decreased.

14. A computer device (10) for adjusting a transformation of a first three-dimensional digital dental model (100) of a first dental structure relative to a second three-dimensional digital dental model (120) of a second dental structure,
the computer device (10) comprising a processing unit (20) and a memory unit (26) storing program instructions (30) executable by the processing unit (20), an execution of the program instructions (30) by the processing unit (20) causing the computer device (10) to:
receive the first three-dimensional digital dental model (100);
receive the second three-dimensional digital dental model (120);
transform the first three-dimensional digital dental model (100), the transforming comprising a positioning of the first three-dimensional digital dental model (100) relative to the second three-dimensional digital dental model (120), the positioning of the first three-dimensional digital dental model (100) relative to the second three-dimensional digital dental model (120) being restricted by a boundary condition (122) defining one or more distance criteria (124) to be preserved between the first and second three-dimensional digital dental model (100; 120), a preserving of the one or more distance criteria (124) by the positioning resulting in a rotating of the first three-dimensional digital dental model (100) around an axis (108) of rotation increasing an angle (114) of a current orientation of the first three-dimensional digital dental model (100) relative to an initial start orientation (110) of the first three-dimensional digital dental model (100) at a start of the rotation until the current orientation reaches a provisional end orientation (112) of the first three-dimensional digital dental model (100) at an end of the rotation with a maximum angle (116) relative to the start orientation (110), a definition of the start orientation (110) being stored for further use; and
adjust the transforming of the first three-dimensional digital dental model (100), a preserving of the one or more distance criteria (124) by the adjusting of the transforming resulting in a further rotating of the first three-dimensional digital dental model (100) around the axis (108) of rotation using the stored definition of the start orientation (110) as a first reference orientation, the further rotating at least partially reversing the rotating of the first three-dimensional digital dental model (100) such that the current angle (114) relative to the start orientation (110) is decreased.

15. A manufacturing system (14) comprising the computer device (10) of claim 14, the manufacturing system (14) further comprising a manufacturing device (60, 70) configured for manufacturing a dental prosthesis (150),
execution of the program instructions (30) by the processing unit (20) further causing the computer device (10) to:
generate a three-dimensional digital prosthesis model (140) of the dental prosthesis (150) comprising the adjusted first three-dimensional digital dental model (100);
provide control data (138) for controlling the manufacturing of the dental prosthesis (150) using the manufacturing device (60, 70), the control data (138) defining the three-dimensional digital prosthesis model (140); and
control the manufacturing device (60, 70) to manufacture the dental prosthesis (150) using the control data (138), the dental prosthesis (150) being manufactured as a physical copy of the three-dimensional digital prosthesis model (140).
